(19) Europäisches Patentamt
European Patent Office
Office européen des brevets

(11) EP 0 744 125 B1

(12) EUROPEAN PATENT SPECIFICATION

(45) Date of publication and mention of the grant of the patent:
**14.07.1999 Bulletin 1999/28**

(51) Int. Cl.$^6$: **A01N 59/12**, A01N 25/08

(21) Application number: **96303752.8**

(22) Date of filing: **24.05.1996**

(54) **Iodo-complex and its use**

Jodkomplex und dessen Verwendung

Complexe d'iode et son application

(84) Designated Contracting States:
**DE FR GB**

(30) Priority: **25.05.1995 JP 16677395**
**08.12.1995 JP 32002095**

(43) Date of publication of application:
**27.11.1996 Bulletin 1996/48**

(73) Proprietors:
- **MIZUSAWA INDUSTRIAL CHEMICALS, LTD.**
**Chuo-ku Tokyo (JP)**
- **NIPPOH CHEMICALS CO., LTD.**
**Chuo-ku Tokyo (JP)**

(72) Inventors:
- **Tanaka, Masanori,**
**c/o Mizusawa Ind. Chem., Ltd.**
**Tokyo (JP)**
- **Abe, Kiyoshi,**
**c/o Mizusawa Ind. Chem., Ltd.**
**Tokyo (JP)**
- **Takai,Kiyoshi,**
**c/o Mizusawa Ind. Chem., Ltd.**
**Tokyo (JP)**
- **Watanabe, Kiyoshi,**
**c/o Mizusawa Ind. Chem., Ltd.**
**Tokyo (JP)**

(74) Representative:
**Cresswell, Thomas Anthony**
**J.A. KEMP & CO.**
**14 South Square**
**Gray's Inn**
**London WC1R 5LX (GB)**

(56) References cited:
**DE-A- 2 611 701**
**US-A- 5 043 090**
**US-A- 5 342 876**

- **DATABASE WPI Section Ch, Week 9127 Derwent Publications Ltd., London, GB; Class B06, AN 91-196387 XP002024867 & JP 03 120 204 A (MIZUSAWA CHEMICAL IND KK) , 22 May 1991**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7/3.6

## Description

BACKGROUND OF THE INVENTION

(Field of the Invention)

The present invention relates to an iodo-complex having a sustained release ability for iodine. More specifically, the invention relates to an iodo-complex which incorporates iodophor in an inorganic porous carrier, suppresses odor specific to an iodine compound and gradually releases effective iodine into an aqueous medium or into the open air, and to an antibacterial agent comprising the iodo-complex as a chief component and having a broad antibacterial spectrum.

(Description of the Prior Art)

Conventional disinfectants and antibacterial agents can be represented by chlorine-type agents such as chlorine, hypochlorite, chloramine T, and the like; iodine-type agents; cationic surfactant-type agents; phenol-type agents such as cresol and the like; aldehyde-type agents such as formalin and the like; biguanide-type agents such as gluconate agent, chlorohexidine hydrochloride salt and the like; alcohol-type agent; and metals such as copper, zinc, silver and the like as well as compounds thereof, exhibiting their respective features.

Among them, as is well known, iodine exhibits excellent antibacterial property to a degree comparable with that of halogen-containing hypochlorous acid, and is relatively safe for human body and has, hence, been widely used as a disinfectant until now. For sterilizing metallic utensils such as scissors, razors, hair clippers, etc. used in barbers and beauty parlors businesses, use is made of a soft material such as cloth, paper or synthetic resin sheet impregnated with or containing iodine as a cloth for disinfection and for preventing rust. A paste containing iodine has been used as an wound-treating ointment. Furthermore, a carrier such as activated carbon, zeolite, diatomaceous earth or calcium silicate carrying or occluding iodine has been used for such applications as cleaning the air, removing odor, controlling bacteria and virus.

Japanese Laid-Open Patent Publication No. 237171/1993 discloses a foamed polyurethane impregnated with a sterilizer composition which comprises a medium such as alcohol or ether containing iodine, iodide ions and a nonionic surfactant, and teaches that this foamed polyurethane is useful as a cleaning material for surgery operations. Japanese Laid-Open Patent Publication No. 174632/1992 discloses a wet tissue impregnated with an iodine-type sterilizer composition containing iodine, alcohol, polyol and polyvinyl pyrrolidone. Furthermore, Japanese Laid-Open Patent Publication No. 40048/1985 discloses a sanitary napkin obtained by impregnating an absorbing material with povidone iodo as a sterilizing agent.

U.S. Patents Nos. 4,911,859 and 5,043,090 disclose a cleaner and a sanitizer for use in toilets, comprising a gypsum occluding iodophor such as povidone iodo in the form of tablets using, as a binder, a polyethylene oxide copolymer having a molecular weight of 10,000 to 60,000, PEG-8000 or a PEG derivative.

Japanese Laid-Open Patent Publication No. 169529/1992 discloses a combination of chitosan which is a chief component, povidone iodo, gluconate and hypochlorite as an agent for preventing inflammation of udders of cattle.

Iodine has such advantages as strong antibacterial activity, broad antibacterial spectrum and safety accompanied, however, by such problems as odor and color specific to iodine and difficult handling. Iodophor, on the other hand, has such advantages as suppressed odor of iodine and easily handling yet exhibiting the same antibacterial activity and antibacterial spectrum same as those of iodine. However, the effect is lost within relatively short periods of time, and sustenance of antibacterial activity is not yet satisfactory.

When the iodophor is carried by a variety of carriers, furthermore, the interaction takes place between the carrier and iodine, and the amount of effective iodine decreases.

SUMMARY OF THE INVENTION

The object of the present invention, therefore, is to provide an iodo-complex which suppresses odor specific to iodine, stably maintains the amount of effective iodine for extended periods of time and sustains antibacterial activity, and to provide an antibacterial agent using the iodo-complex.

Another object of the present invention is to provide toilet sand for pets having urea-absorbing property, solidifying property, deodoring activity, and environmental sterilizing activity.

A further object of the present invention is to provide a filter capable of antibacterially treating the air contaminated with suspended bacteria for extended periods of time.

A still further object of the present invention is to provide an antibacterial mat capable of removing bacteria adhered to the bottoms of the shoes, so that bacteria can be prevented from being brought indoors for extended periods

of time.

A yet further object of the present invention is to provide a thermoplastic polymer composition in which antibacterial activity is stably maintained in a thermoplastic polymer or in an elastomer, making it possible to produce molded articles that exhibit excellent antibacterial activity in a sustained manner.

According to the present invention, there is provided an iodo-complex having a sustained release ability for iodine, comprising (A) an inorganic carrier having a BET specific surface area of from 80 to 800 $m^2/g$ and a pore volume of from 0.2 to 2.3 ml/g as measured by the nitrogen adsorption method, and (B) an iodophor carried by said inorganic carrier.

There is no particular limitation on the inorganic carrier (A) provided it has the above-mentioned specific surface area and pore volume. However, particularly preferred examples include a powder of porous and amorphous silica and, particularly, the powder having a pore volume with a pore radius of from 7.5 to 150 angstroms of from 20 to 90% per the whole pore volume; a powder of porous activated alumina or activated silica-alumina; a powder of phyllosilicate or aluminophyllosilicate and, particularly, clay mineral of the smectite group; a powder of bentonite or a mixture of bentonite and aluminum sulfate; a powder of fraiponite or a porous amorphous silica carrying fraiponite on the surfaces thereof; and a powder of hormite-type clay minerals. It is desired that the inorganic carrier (A) has an average particle diameter of from 0.0001 to 4 mm.

Another preferred example of the inorganic carrier (A) may be a porous molded article comprising at least the one selected from the group consisting of amorphous silica, activated alumina, activated silica-alumina, clay minerals of the smectite group, acid-treated clay minerals of the smectite group, fraiponite, and hormite-type clay minerals.

A further example of the inorganic carrier (A) is an amorphous and porous silica of the form of spherical particles having a volume-based median diameter of from 10 to 100 $\mu m$ as measured by using the Coulter counter, an apparent density (in compliance with JIS K-6220) of from 0.2 to 0.5 ml/g, and a pore volume with a pore radius of from 18 to 43500 angstroms of from 0.5 to 3.5 ml/g as measured by the mercury intrusion porosity method. It is desired that the spherical amorphous silica particles have meso pores of a pore radius of from 75 to 1000 angstroms as measured by the mercury intrusion porosity method and a pore volume of not smaller than 0.2 ml/g.

A still further example of the inorganic carrier (A) is an amorphous silica in the form of spherical particles having a primary particle diameter of from 0.2 to 20 $\mu m$ as observed using a scanning electron microscope, the individual particles being obviously spherical particles having a degree of sphericity of not smaller than 0.9, and further having an apparent density (in compliance with JIS K-6220) of from 0.1 to 0.5 ml/g, a pore volume of from 0.5 to 2 ml/g as measured by the nitrogen adsorption method, and an oil-absorbing amount of from 100 to 400 ml/100 g.

It is desired that the iodophor (B) contains effective iodine in an amount of from 5 to 20% by weight. For this purpose, it is particularly desired to use povidone iodo, i.e., polyvinylpyrrolidone-iodine complex.

It is desired that the iodophor (B) is contained in an amount of from 0.05 to 30 parts by weight and, particularly, from 0.1 to 20 parts by weight per 100 parts by weight of the inorganic carrier (A).

According to the present invention, there is further provided an iodo-complex having a sustained release ability for iodine, comprising (A) an inorganic carrier having a BET specific surface area of from 80 to 800 $m^2/g$ and a pore volume of from 0.2 to 2.3 ml/g as measured by the nitrogen adsorption method, (B) an iodophor carried by said inorganic carrier, and (C) a covering layer for covering them and comprising at least one of oil, wax or resin.

It is desired that the iodine compound (B) is contained in an amount of from 0.05 to 30 parts by weight per 100 parts by weight of the inorganic carrier (A), and the covering layer (C) is contained in an amount of from 1 to 30 parts by weight per 100 parts by weight of the total of (A) and (B).

According to the present invention, there is provided an antibacterial agent comprising the above-mentioned iodo-complex which gradually releases iodine.

According to the present invention, there is further provided toilet sand for pets containing the above-mentioned iodo-complex which gradually releases iodine.

According to the present invention, furthermore, there is provided a filter for an air cleaner containing the above-mentioned iodo-complex which gradually releases iodine.

According to the present invention, there is further provided a thermoplastic polymer composition obtained by blending a thermoplastic resin or an elastomer with the above-mentioned iodo-complex which gradually releases iodine. According to this embodiment, it is desired that the iodo-complex is blended in an amount of from 0.1 to 30 parts by weight per 100 parts by weight of the thermoplastic resin or the elastomer. This thermoplastic polymer composition is convenient for a carpet or a mat.

According to another embodiment of the present invention, there is provided an antibacterial filter comprising at least one layer of sheet obtained by filling an air-permeable woven fabric or nonwoven fabric of inorganic fibers, animal fibers, plant fibers, or synthetic resin fibers with the above-mentioned iodo-complex.

According to a further embodiment of the present invention, there is provided an antibacterial mat comprising at least one layer of sheet obtained by filling a woven fabric or nonwoven fabric of inorganic fibers, animal fibers, plant fibers, or synthetic resin fibers with the above-mentioned iodo-complex.

BRIEF DESCRIPTION OF THE DRAWINGS

Fig. 1 is a diagram illustrating an apparatus for testing antibacterial activity of a filter used for air cleaners;
Fig. 2 is a perspective view illustrating an antibacterial sheet;
Fig. 3 is a sectional view of the antibacterial sheet along the line A-A; and
Fig. 4 is a diagram illustrating a state where the antibacterial sheet is being used.
Fog. 5 is a diagram illustrating a width of blocking band against bacteria in the antibacterial testing.

1: air pump
2: gas pipe
3: container
4: rubber plug
5: glass tube
6: used animal-breeding chips
7: sample (GI-9) )
8: control culture medium
9: testing culture medium
10: cotton plug
11: protruded portion
12: upper sheet having protruded and recessed portions
13: flat lower sheet
14: sample
15: patient
16: sheet
17: antibacterial sheet
18: mattress
21: laboratory dish (schale)
22: sample
23: width of the propagation blocking band against bacteria (mm)
24: colony of bacteria

DETAILED DESCRIPTION OF THE PREFERRED EMBODIMENTS

The present invention has a feature in that an iodophor is selected as iodine agent, and is combined with an inorganic carrier having a BET specific surface area of from 80 to 800 $m^2$/g and a pore volume of from 0.2 to 2.3 ml/g as measured by the nitrogen adsorption method.

The iodophor is a complex of iodine using a surfactant or a polymer as a carrier and exhibits strong antibacterial activity and a broad antibacterial spectrum but does not produce offensive odor specific to iodine.

Like the element iodine, the iodophor sterilizes microorganisms such as bacteria, viruses, fungi, spores, protozoa and yeast, and further works to control some kinds of insects, parasites and nematodes, offering an advantage that the microorganisms being controlled do not acquire immunity. Moreover, the iodophor is safer and easier to use than the traditional halogen-type antibacterial agents, and little stimulates man and animals.

A second feature resides in that the above-mention ed iodophor is carried by an inorganic porous powder having a BET specific surface area of from 80 to 800 $m^2$/m and a pore volume of from 0.2 to 2.3 ml/g.

The iodophor that is carried by the inorganic porous powder exhibits improved stability when it is used as an antibacterial agent, gradually releases iodine and sustains sterilizing activity for longer periods of time.

The iodophor such as polyvinyl pyrrolidone-iodine complex assumes a powdery form and, by itself, remains stable for a relatively long period of time even in the form of an aqueous solution obtained by dissolving it in water. When used as an antibacterial agent, however, the iodophor loses the sterilizing activity within relatively short periods of time upon coming in contact with organic matters such as sugar, amino acid, lipids, proteins and the like.

The inorganic porous powder used in the present invention does not substantially exhibit interaction to iodine, stably carries effective iodine, exhibits surface activity and contains pores therein. Therefore, the iodophor is stably held in the inorganic porous powder and gradually elutes out of the inorganic porous powder. Thus, iodophor or iodine is gradually released and sterilizing activity is sustained.

[Iodophor]

According to the present invention, any widely known iodophor preparation can be used as the iodophor. The iodophor is a complex which exhibits sterilizing activity but does not produce offensive odor specific to iodine. In this complex, iodine is incorporated in the carrier of a polymer or a surfactant.

In the case of a povidone iodo, a polyvinyl pyrrolidone and molecular iodine are forming a complex as represented by the following structural formula (1),

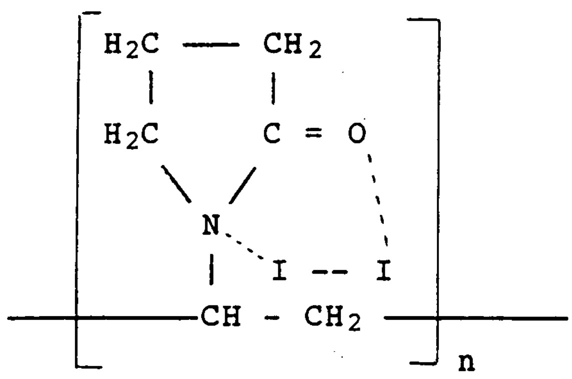

Examples of the polymer for carrying the iodophor include polyether glycol, polyvinyl alcohol, polyacrylic acid, polyamide and polyoxyalkylene in addition to polyvinyl pyrrolidone. Examples of the surfactant carrier may include those having anionic property, cationic property, amphoteric property, and nonionic property. They can be used in a single kind or in a combination of two or more kinds, such as, in a combination of a polymer and a surfactant.

Concrete examples of the iodophor preparation include povidone iodo (polyvinyl pyrrolidone-iodine complex), iodine-alkylpolyether alcohol complex (G. S.I.), polyethoxypolypropoxypolyethoxyethanol-iodine complex (locline), nonylphenoxypolyethoxyethanol=iodine complex, polyoxyethylene-added plant oil-iodine complex, polyoxyethylene-added fatty acid-iodine complex, polyoxyethylene-added fatty alcohol-iodine complex, fatty acid amide-iodine complex, quaternary organoammonium-iodine complex, and the like. Among them, however, povidone iodo is preferred.

The iodophor containing effective iodine in an amount of from 0.005 to 3% and, particularly, from 0.01 to 2% is advantageous from the standpoint of antibacterial activity exhibited in a sustenained manner. When the amount of effective iodine is smaller than the above-mentioned range, the antibacterial activity is not sufficient. When the amount of effective iodine is not smaller than the above-mentioned range, on the other hand, stability is lost and offensive odor is produced.

The iodophor is available in the form of either a liquid or a powder. Though the iodophor of either form can be used in the present invention, the powder is advantageous from the standpoint of adjusting the amount of water.

The iodophor represented by the povidone iodo has the following advantages:

(1) The iodophor can be easily dissolved in cold water or in ethyl alcohol. An aqueous solution containing 10% of povidone iodine which is a usually used concentration remains stable and does not undergo a change for a year when kept at room temperature. The aqueous solution in which the polyvinyl pyrrolidone and iodine are forming a complex exhibits a pH of from 2 to 3.5.

(2) The iodophor exhibits antibacterial activity against bacteria, true fungi (mold, yeast), viruses, some kinds of insects, protozoa and parasites, and exhibits strong antibacterial power by which many microorganisms are annihilated within several seconds to several minutes. Not so many antibacterial agents exhibit excellent activity against true fungi.

(3) The microorganisms do not acquire immunity against pivodone iodo. Therefore, the iodophor exhibits antibacterial activity effectively even against immunized bacteria that cannot be controlled by antibiotics. For instance, MRSA is immune to almost all antibiotics.

(4) The iodophor does not almost stimulate the tissues of mammals, does not stimulate or does not cause pain even to an wound or mucous membrane, and can hence be easily treated.

(5) The iodophor exhibits strong sterilizing effect irrespective of gram positive or gram negative bacteria. Like chlorine, furthermore, the iodophor exhibits strong sterilizing power even against true fungi that could not be accomplished by surfactants, biguanide derivatives or phenol-type sterilizing agents.

(6) Against bacterial spores, the iodophor must be brought into contact at a concentration of as high as 100 to 500

ppm for extended periods of time. No sterilizer other than chlorine or the aldehyde-type one is capable of annihilating bacterial spores at normal temperature. In addition to the povidone iodo, chlorine and aldehyde only are capable of sterilizing spores at normal temperature.

The iodophor is excellent in that it exhibits less toxicity and little stimulates man and animals. Described below are the toxicities caused by the pivodone iodine which is a representative example of iodophor.

1) Acute aural toxicity.

By using 12 groups of rats each group consisting of 10 rats, an aqueous solution of the pivodone iodo (concentration of effective iodine, 2.5%) was evaluated for its acute aural toxicity. LD50 (two weeks after) was 1.300 mg/kg in terms of the amount of effective iodine (Journal of the International College of Surgeons 25, 727 (1956)).

2) Effect against healthy skin.

A povidone iodo solution (10%) was applied to the skins of 200 persons for 96 hours by a patch testing method but no abnormal condition was recognized (Journal of the International College of Surgeons 25, 727 (1956)).

3) Effect against injured skin.

The human skin or the hare skin was abraded using a coarse sand-paper, and a povidone iodo solution (10%) was applied thereto by the patch testing method. Like the healthy skin, no abnormal condition was recognized. No infection took place at that part, and the abrasion was cured (Journal of the International College of Surgeons 25, 727 (1956)).

4) Effect to the eyes.

0.5 cc of a 10% povidone iodine solution was dropped to the eyes of 25 hares to observe the eyes. Conjunctivae became reddish to some extent which, however, disappeared within a few days. 0.1 cc of the 10% povidone iodo solution was dropped to the eyes of 25 hares and to the eyes of 25 guinea pigs everyday for 15 days, and red spots appeared temporarily which, however, disappeared after several hours. No abnormal condition was recognized during the testing period of 15 days (Journal of the International College of Surgeons 25, 727 (1956)).

5) Effect to the throat.

The povidone iodo solution (10%) was applied to the throats of 25 volunteers, and 60% of them felt sharp stimulation for 10 to 20 minutes. Mucous membranes in the throats became reddish to some extent which, however, disappeared in about two hours. No abnormal condition was recognized in 40% of persons.

The povidone iodo solution was applied to the throats of 10 persons for 15 days, and no abnormal condition was recognized in five of them. Five other persons showed reddish color to some extent at first which, however, was not worsened any more. Two weeks later, the same persons were put to the same test and quite the same results were obtained (Journal of the International College of Surgeons 25, 727 (1956)).

6) Effect to diathetic persons.

The povidone iodo and Lugol's solution were applied to the skins of three persons allergic to iodine. The skins to which the Lugol's solution was applied developed specific hypersensitiveness but the povidone iodine did not cause anybody to develop abnormal condition (Journal of the International College of Surgeons 25, 727 (1956)).

The povidone iodine was used as a sterilizer for external application for 5900 persons over three years, and only two persons developed diathesis due to this sterilizer. These two persons, however, did not develop systemic toxic symptoms or hypersensitiveness to iodine (The Bulletin American Society of Hospital Pharmacists 13, 226 (1956)).

[Inorganic porous powder]

According to the present invention, the inorganic porous powder for carrying the iodophor has a BET specific surface area of from 80 to 800 $m^2/g$ and a pore volume of from 0.2 to 2.3 ml/g.

It is important that the inorganic porous powder has the above-mentioned BET specific surface area and the pore volume. When the BET specific surface area and the pore volume are smaller than the above-mentioned ranges,

the ability for gradually releasing iodophor or iodine and the sustenance of sterilizing activity become inferior to those of when the BET specific surface area and the pore volume lie within the ranges of the present invention. When the BET specific surface area and the pore volume are greater than the above-mentioned ranges, on the other hand, the strength and abrasion resistance of the particles carrying iodophor decrease.

Any inorganic porous material can be used provided it has the above-mentioned BET specific surface area and the pore volume. Though not necessarily limited thereto only, examples may include amorphous silica, activated alumina, silica-alumina, various silicates, phyllosilicate, aluminosilicate, phylloaluminosilicate, tectoaluminosilicate, etc.

When used being mixed to the thermoplastic polymer or in the form of a powder, the inorganic porous material has a particle diameter of from 0.1 to 100 μm. The inorganic porous material may be used as a molded article of a powder. From the standpoint of handling and preventing the powder from flying, it is desired that the inorganic porous material is in the form of particles. In general, it is desired that the particles have a diameter of from 1 to 10 mm and, particularly, from 2 to 5 mm. The particles may have any shape such as spherical shape, cubic shape, cylindrical shape, prism shape, granular shape, tablet shape or amorphous shape.

A preferred example of the inorganic porous carrier for carrying iodophor may be amorphous silica particles which is either of the gel type or the sedimentation type.

A) Amorphous silica by the instantaneous granulating method.

An example of the former type may be porous spherical silica by the instantaneous granulation method. This silica is obtained by using a two-fluid nozzle, instantaneously mixing together a sodium silicate solution or a sodium silicate solution containing silica hydrogel and sulfuric acid such that the pH when mixed together is from 8 to 9, spraying the mixture into the air so that spherical silica particles are instantaneously gelled, permitting the formed gel to fall down into the lower acidic solution, followed by washing with water and drying. The inorganic porous powder of this type has a particle diameter of from 0.1 to 4 mm, a BET specific surface area of from 300 to 800 $m^2/g$, and a pore volume of from 0.6 to 2 ml/g as measured by the nitrogen adsorption method. The carrier of this type is of a spherical shape having smooth surfaces, exhibits excellent fluidity, can be easily handled without permitting the powder to fly, excellently carries the iodophor inside the particles, and is very useful as a particulate carrier for the antibacterial agents.

B) Amorphous silica by the hydrogel granulation method.

Another amorphous spherical silica of the gel type can be represented by the silica of the hydrogel spray granulation method. This silica is obtained by mixing an alkali silicate aqueous solution and an aqueous solution of acid such as sulfuric acid together and reacting them together such that the pH of the sol is from 2 to 6, gelling the reaction mixture, shredding the obtained gel, followed by washing with water and, as required, by the treatment with hot water at a temperature of from 100 to 150°C, wet-pulverizing the silica hydrogel that is obtained, and spraying, drying and granulating the aqueous slurry of the silica hydrogel.

The amorphous silica of this type is in the form of spherical particles having a volume-based median diameter of from 10 to 100 μm and, particularly, from 25 to 80 μm as measured by the Coultar counter method, and has an apparent density (in compliance with JIS K-6220) of from 0.2 to 0.5 ml/g and a pore volume with a pore radius of from 18 to 43500 angstroms of from 0.5 to 3.5 ml/g as measured by the mercury intrusion porosity method. Besides, the spherical amorphous silica particles have mesopores of a pore radius of from 75 to 1000 angstroms as measured by the mercury intrusion porosity method and a pore volume of not smaller than 0.2 ml/g.

The amorphous silica of this type has large voids in the particles, exhibits excellent property for carrying the iodophor, and is useful as a powdery carrier for the antibacterial agent permitting powder to fly little, and is further useful as a starting material for the molded carrier that will be described later.

C) Amorphous silica by the coagulation growth method.

The spherical amorphous silica by the sedimentation method can be represented by porous and spherical silica or silicate particles obtained by the method disclosed in Japanese Laid-Open Patent Publication No. 193927/1993 filed by the present applicant. This silica is obtained by growing a partly neutralized product of an alkali silicate aqueous solution into spherical particles by using a watersoluble high molecules such as carboxymethyl cellulose as a coagulation growing agent, and neutralizing the spherical particles or further reacting the particles with a hydroxide or a salt of a metal of the Group II of periodic table in an aqueous medium. The spherical particles of the silica or silicate are aggregate of primary silica particles and are porous, carrying the iodophor among the primary particles and exhibiting particularly excellent carrying property.

The amorphous silica has a primary particle diameter of from 0.2 to 20 μm as observed using a scanning-type electron microscope, the individual particles being obviously spherical particles having a degree of sphericity of not

smaller than 0.9, and further having an apparent density (in compliance with JIS K-6220) of from 0.1 to 0.5 ml/g, a pore volume of from 0.5 to 2 ml/g as measured by the nitrogen adsorption method, and an oil-absorbing amount of from 100 to 400 ml/100 g.

The amorphous silica of this type can be used in its own form or being modified into the form of an amorphous silicate or a fine crystalline silicate layer. Examples of the metal for forming a silicate include zinc, magnesium, calcium and the like. To synthesize the spherical silicate, spherical particles composed of a partly or a completely neutralized product of alkali silicate obtained by the above-mentioned method and a hydroxide of magnesium, calcium or zinc or one or two or more kinds of hydroxides of inorganic acid salts such as nitrates, chlorides or sulfates, or salts thereof, are reacted together in the presence of an aqueous medium, in order that a magnesium phyllosilicate, a calcium phyllosilicate or a zinc phyllosilicate is formed on at least the surfaces of the spherical particles.

D) Phylloaluminosilicate.

As the phylloaluminosilicate, there may be used a variety of natural or synthetic clay minerals or chemically modified products thereof. Examples include clay minerals of the smectite group such as montmorillonite, bentonite, beidellite, nontronite, saponite, hectorite, sauconite and stevensite; clay minerals of the kaolin group such as metakaolin, halloysite, metahalloysite and antigorite; as well as sepiolite, palygorskite, and vermiculite. Examples of the chemically modified product include activated clay obtained by activating montmorillonite such as acidic clay by the treatment with an acid and activated bentonite obtained by activating montmorillonite such as acidic clay by the treatment with an alkali.

There can be further favorably used activated bentonite containing aluminum sulfate or activated clay.

The phylloaluminosilicate and modified products thereof of this type not only serve as excellent iodophor carriers but also exhibit deodoring activity and coagulating property, and are useful as toilet sand for pets, environmental sterilizers, etc.

E) Chain clay minerals

Sepiolite and palygorskite of the hormite type pertaining to a chain clay mineral have appearance of fibers or converged body thereof, and its fundamental structure consists of 3-octahedral magnesium silicate clay minerals comprising chiefly magnesium that forms a skeleton of an octahedral layer. The magnesium silicate clay mineral has a three-dimensional chain crystalline structure, and voids formed in the gaps of the fibers and in the converged body thereof forming the appearance have a BET specific surface area of from 100 to 600 $m^2/g$. Besides, this magnesium silicate clay mineral has a converged structure and is porous exhibiting adsorptive action. Therefore, this mineral exhibits excellent properties for carrying iodophor and for removing odor, and can be easily blended into a variety of fibers.

Prior to using these clay minerals as a carrier of the present invention, it is desired to subject the clay minerals to the primary pulverization such as shredding using a ball mill or a hammer mixer, knead-pulverization using a supermixer or a mixing and grinding machine, or shock pulverization using atomizer or jet mill. In other words, it is desired to use the clay minerals for carrying the iodophor after the chain structure of the converged body and the layer structure of the scales are partly frayed and untied.

F) Fyllosilicate.

As an inorganic powder, there can be used a fine crystalline synthetic zinc phyllosilicate having a large specific surface area, a magnesium phyllosilicate, an aluminum-containing zinc phyllosilicate or an aluminum-containing magnesium phyllosilicate. The phyllosilicates exhibit excellent deodoring activity and make it possible to accomplish excellent deodoring activity as well.

Examples of the particularly preferred phyllosilicate may include (1) a zinc phyllosilicate and an aluminum-containing zinc phyllosilicate having an X-ray diffraction image of the fraiponite type and a BET specific surface area of 100 $m^2/g$ as disclosed in Japanese Laid-Open Patent Publication No. 10021/1986, and (2) a composite phyllosilicate, i.e., amorphous and porous silica or silica-alumina, and zinc or magnesium phyllosilicate, or aluminum-containing zinc or magnesium phyllosilicate formed on the surfaces of the primary particles thereof as disclosed in Japanese Patent Publication No. 79602/1993.

G) Molded articles.

According to the present invention, the porous powder that is molded can be used as an inorganic carrier. According to this embodiment, there is preferably used a porous molded article composed of at least the one selected from the group consisting of amorphous silica, activated alumina, activated silica-alumina, clay minerals of the smectite

group, acid-treated clay minerals of the smectite group, fraiponite and clay minerals of the hormite type. The molding method may be any widely known granulation method such as compression molding method, tablet molding method, rolled granulation method, spray granulation method or extrusion granulation method.

Described below is a compression molded article having a ring shape. Among the above-mentioned powders for this application, the amorphous silica (B) mentioned above is particularly suited. This amorphous silica has an excellent moldability and does not need forming assistants such as binder, lubricant or the like agents. Here, however, the lubricant reduces the internal friction and external friction of the starting powder that is to be molded, making it possible to improve filling property and compression property and, particularly, works to suppress the concentration of stress upon the molded article from the external side when the compression is discontinued and the molded article is taken out. For this purpose, the lubricant is added to the starting material usually in an amount of from about 0.5 to about 1% prior to molding tablets. The lubricant may be talc, starch, magnesium stearate, silicone oil, fluidized paraffin, or polyethylene glycol. According to the present invention, magnesium stearate or silicone oil is used.

The particles are so granulated that the spherical silica particles having a volume-based median diameter of from 25 to 80 μm exist in an amount of from 80 to 95% by volume in the whole spherical silica particles. To 50 kg of spherical silica particles is added 0.5 kg of magnesium stearate and is well mixed together in a rotary drum mixer. The mixture is then fed from a hopper and is molded under a condition in which a turn table rotates at a speed of from 10 to 15 rpm.

In this embodiment, a ring-like porous silica molded article was prepared using a rotary powder molding machine (rotary tabletting machine), Correct D555-C15R, manufactured by Kikusui Mfg. Co. The rotary tabletting machine has a molding mechanism in which mortars are buried in a horizontally rotating turn table along the same circumference thereof maintaining an equal distance, wherein while the turn table rotates, the starting material is fed into the mortars which are then compressed by pestles and are molded by the pestles continuously in a series of operation. The lower pestle is fitted to the mortar and moves together with the mortar. The upper pestle is located in concentric with the mortar and rotates together with the mortar. The upper pestle and the lower pestle move up and down to carry out a series of operations, so that molded articles of silica are obtained. A maximum molding diameter may be from 3 to 15 mm, and a maximum height of filling may be from 3 to 15 mm. In this embodiment, however, ring-like molded articles of porous silica having an outer diameter of 5 mm, an inner diameter of 2 mm and a height of 6 mm are continuously produced under a condition of a compression pressure of 30 $kg/cm^2$ and are used as a molded carrier.

The molded article according to the present invention is in no way limited to the above-mentioned ring-shaped one as a matter of course, and may be any one of, for example, round shape, triangular shape, square shape, rectangular shape, hexagonal shape, ring shape or go stone shape depending upon the kind of the metal mold which consists of a combination of a cylinder (mortar) and upper and lower pistons (upper and lower pestles). By using the rotary tabletting machine, furthermore, it is made possible to provide articles having a maximum diameter of generally from 3 to 30 mm.

[Iodo-complex and its preparation]

The iodophor can be supported on the inorganic porous powder without requiring any particularly cumbersome operation; i.e., the solution of iodophor is applied onto the inorganic porous powder by spray method or impregnation method, and is dried. The solution of iodophor is usually an aqueous solution having a concentration of from 1 to 50% by weight. The drying is effected at a temperature of not higher than 160°C, preferably, not higher than 120°C and, more preferably, not higher than 80°C. The iodophor is carried by the inorganic porous powder in an amount of from 0.05 to 30 parts by weight per 100 parts by weight of the inorganic porous carrier though it may vary depending upon the use. This means is effective in carrying the iodophor by a carrier that has been molded in advance.

Other means may be employed to carry the iodophor on the inorganic porous carrier. That is, the iodophor is mixed into a powder that is a starting material of the inorganic porous carrier, which is then molded into particles or any other shapes to prepare an iodo-complex of the present invention. The iodophor may be mixed in the form of either a powder or a solution. The particles to be molded may be blended with waxes or low-melting resins that will be described later as granulating media.

The above-mentioned components can be mixed or kneaded together by using, for example, a double-screw kneader, a mixing and dipping machine, a sand grinder mill, Atritor, an atomizer, Nara-type pulverizer, a disk vibration mill, a vibration ball mill, Henschel's mixer, a super-mixer, or a combination thereof. The granulation can be carried out by using any widely known means such as extrusion granulation, tabletting, rolling granulation, spray granulation or pulverization granulation.

In order that the iodophor is gradually released from the carrier, the inorganic porous powder or molded article carrying the iodophor may be treated with an organic treating agent such as silicone oil to form a covering layer thereon.

As the organic components, there can be favorably used silicone oil as well as waxes or low-melting resins. It is desired that the organic treating agents are used in amounts of from 1 to 30 parts by weight per 100 parts by weight

of a total of the inorganic porous powder and the iodophor.

Examples of the wax and low-melting resin which are organic components include:

(1) Fatty acid and metal salts thereof such as higher fatty acid (with 8 to 22 carbon atoms) or metal salts thereof (alkaline metal salts, alkaline earth metal salts, Zn salt, Al salt);
(2) Amide, amine and fatty acid esters of monohydric and polyhydric alcohols such as higher fatty acid amide, erucic acid amide, stearyl erucamide, 2-stearamideethyl stearate, ethylenebis fatty acid amide, fatty acid diethanolamine, n-butyl stearate, glycerin fatty acid ester, pentaerythritol, polyethylene glycol fatty acid ester, polyethylene glycol distearate, polyethylene glycol dilaurate, diethylene glycol stearic diester, triglyceride wax, polyethylene wax, and epoxy-modified polyethylene wax; and
(3) Low-melting resins such as epoxy resin having a melting point or a softening point of from 40 to 160°C, xylene-formaldehyde resin, styrene resin, alkyd resin, ethylene-vinyl acetate copolymer, low-melting acrylic resin, polyvinyl butyral, terpene resin and petroleum resin.

These waxes and low-melting resins can be used alone or in a combination of two or more kinds.

To increase the sterilizing effect and to emphasize the production of immediate effect, sterilizing components that immediately produce effect such as potassium iodide/iodine may be added to the iodophor-carrying inorganic porous powder in an amount of not larger than 50 parts by weight per 100 parts by weight of the total of the iodophor and the inorganic porous powder.

[Antibacterial agent]

The iodo-complex of the present invention is useful as an antibacterial agent or an environmental sterilizer (sanitizer) gradually releasing effective iodine. That is, with the iodophor that easily dissolves in water but lacks stability being carried by the inorganic porous carrier, it becomes possible to stably maintain effective iodine. As will become obvious from Example 2 appearing later, furthermore, iodine is released gradually and in a sustained manner.

The iodo-complex which gradually releases iodine obtained by the present invention was used as an antibacterial agent and was tested for its antibacterial activity. As will become obvious from Example 3 appearing later, it is observed that numerous colonies of MRSA bacteria are propagating on the surface of an agar culture medium. Around the iodo-complex pellets placed on the agar culture medium, however, there have been formed so-called propagation blocking bands blocking the propagation of bacteria. The fact that the propagation blocking bands are stably observed for extended periods of time proves the action of the present invention in that effective iodine is gradually released in a sustained manner.

For the purpose of comparison, a povidone iodine powder or an iodo-complex powder of the present invention was spotted on the agar culture medium containing starch. A blue color due to the iodine-starch reaction was confirmed surrounding the powder. On the next day, however, both blue color and brown color of the powder disappeared. When the iodo-complex which gradually releases iodine of the present invention was used, however, blue color could be seen around the powder even on the next day. It will therefore be obvious that the iodo-complex of the present invention exhibits both stability and ability to gradually release iodine in combination.

The iodo-complex of the present invention exhibits a broad antibacterial spectrum like that of the iodophor represented by povidone iodine.

Described below are known data of povidone iodo. That is, the iodo-complex of the invention exhibits antibacterial effect (annihilated in one minute with a 0.01% aqueous solution) for the bacteria (S. aureus, E. Faecalis, E. coli, K. pneumoniae, S. marcescens, P. mirabilis, P. aeruginosa), exhibits antibacterial effect (annihilated in five minutes with a 0.005% aqueous solution) for the true fungi (C. albicans-type A, -B, C. tropicalis, C. stellatoidea, C. quilliermondii, C. parapsilosis, C. krusei, T. glabrata, Cr. neoformans), and exhibits antivirus effect (virus infection value of $10^{-2.8}$ to $10^{-1}$ in 15 to 60 seconds with a 0.1 to 0.02% aqueous solution) for the viruses (herpes simplex, influenza virus, ECHO virus, human immunodeficiency virus (HIV), coxsacki).

In addition to the above, the iodo-complex of the invention exhibits antibacterial activity even for the following bacteria (gram negative bacteria, gram positive bacteria, acid-fast bacteria, true fungi, viruses, protozoa); i.e.,

(1) Gram negative bacteria such as Aerobacter aerogenes, Aeromonas liquefaciens, Achromobacter xylosoxidans, Chlamydia trachomatis, Citrobacter freundii, Enterobacter aerogenes, Enterobacter cloacae;
(2) Gram positive bacteria such as Bacillus cereus, Bacillus pumilis, Bacillus subtilis, Clostridium histolyticum, Clostridium perfrinoens, Clostridium septicum, Clostridium tetani, Corynebacterium acnes;
(3) Acid-fast bacteria such as Mycobacterium fortuitum, Mycobacterium tuberculosis;
(4) True fungi such as Aspergillus flavus, Aspergillus niger, Aspergillus fumigatus, Aspergillus terreus, Pityrosporum ovale, Trichophyton rubrum, Rhodotorula glutinis, Trichophyton interdigitale;

(5) Viruses such as Adenovirus, Cytomegalovirus, Influenza Virus A, Herpes simplex Types 1 & 2, Rabies Virus, Rubella Virus, Vaccinia Virus; and

(6) Protozoa such as Treponema pallidum; Entamoeba histolytica, Trichomonas vaginalis.

The antibacterial agent and the environmental sterilizer of the present invention can be applied to any place where antibacterial activity and environmental sterilizing activity are required in any form such as powder, granules or molded articles. The antibacterial agent and the environmental sterilizer can be applied in the following ways. That is, a powder may be directly sprayed in the required places, or the powder or the molded article is contained in a container which permits the passage of the air or the water, and the container is placed on a place where antibacterial activity and environmental sterilizing activity are required.

The required amount may differ depending upon the required degree of antibacterial activity or sterilizing activity. Generally, however, effective iodine should be supplied in an amount of from 1 to 200 ppm and, particularly, from 10 to 100 ppm.

[Composition of toilet sand for pets]

According to an embodiment of the present invention, the iodophor is supported by the granular bentonite having a BET specific surface area of from 80 to 800 $m^2/g$ and a pore volume of from 0.2 to 2.3 ml/g in an amount of from 0.05 to 20 parts by weight and, particularly, from 0.1 to 10 parts by weight in average per 100 parts by weight of the granular bentonite, to obtain toilet sand for pets. When the amount of the iodophor is smaller than the above-mentioned range with respect to the bentonite, antibacterial activity is not exhibited to a sufficient degree. When the amount of the iodophor is larger than the above-mentioned range, on the other hand, solidifying property is not obtained to a sufficient degree.

As a base agent of toilet sand for pets, there is usually used natural or synthetic bentonite in a granular form. Even the present invention uses the natural or synthetic bentonite in a granular form as the base agent. It is desired that the granular bentonite usually has a particle size of from 1 to 10 mm and, particularly, from 2 to 5 mm. The particles may have any shape such as spherical shape, cubic shape, cylindrical shape, prism shape, granular shape, tablet shape or amorphous shape. A preferred bentonite is an activated bentonite as disclosed in Japanese Patent Publication No. 73689/1993 containing montmorillonite as a chief component and cristobalite as a minority component, having a chemical composition as expressed by molar ratios of,

$Al_2O_3/SiO_2$ = 0.095 to 0.16
$Na_2O/SiO_2$ = 0.8 x $10^{-2}$ to 4.5 x $10^{-2}$
$MO/SiO_2$ = 4.5 x $10^{-2}$ to 9.5 x $10^{-2}$

(wherein M is an alkaline earth metal),

and a degree of swelling of not smaller than 30 ml/2 g as measured by the ACC method and a flow behavior index (n) of not larger than 0.53.

The degree of swelling is measured according to the ACC method as described below.

Into a 100-ml messcylinder with a plug containing 100 ml of ion-exchanged water is added 2 g of the sample (water content of 4 to 10.0%) being divided into about 10 times in a manner that it does not almost adhere to the inner walls. The following sample is added after the previously added sample has almost settled. After the sample is all added, the plug is fitted to the cylinder which is then left to stand for 24 hours to read the apparent volume of the sample that has deposited in the container. The swelling force is expressed in a unit of ml/2 g.

The activated bentonite is produced by adding a solid sodium carbonate in an amount of 1 to 5% by weight reckoned as an anhydride to the water-containing acidic clay having a pH of from 3 to 7 as measured as a saline water slurry of a concentration of 3 g/100 ml and containing cristobalite, kneading them together at a temperature of not lower than 50°C, and converting the acidic clay into the activated bentonite under a condition where cristobalite is still remaining.

The activated bentonite is particularly suited for use as a base agent of toilet sand for pets since it absorbs urea of pets and solidifies enabling the solidified portion to be easily taken out and absorbing odor to some extent.

Other excellent examples of the base agent of toilet sand for pets include a granular bentonite containing aluminum sulfate and clay minerals of the smectite group treated with an acid. Among the granular bentonites containing aluminum sulfate, it is particularly desired to use the one containing aluminum sulfate in an amount of from 5 to 30% by weight reckoned as granular bentonite. As the granular bentonite containing aluminum sulfate, there can be used a mixture of the granular bentonite to which is added aluminum sulfate in the form of a powder, granules or aqueous solution in such an amount that the amount of aluminum sulfate is 5 to 30% by weight with respect to the activated bentonite, or a molded article thereof. Aluminum sulfate contained in the bentonite works to enhance the properties for being molded

into granules, for absorbing urea of pets and for being solidified, and for enhancing antibacterial activity of the iodophor.

Among the acid-treated smectite clays, furthermore, the one containing a salt of aluminum in an amount of 1 to 25% by weight reckoned as $Al_2O_3$ can be preferably used. As the acid-treated smectite clay, there can be used a powder or a molded article of the powder obtained by reacting an acidic clay and/or an acid-treated product thereof directly with a watersoluble aluminum salt in the presence of an aqueous medium, and drying or firing the product thereof.

According to this embodiment, part or whole of the granular bentonite needs carry the iodophor in small amounts in average as described above. Advantageously, therefore, the iodophor solution can be carried by a spray method. By spraying the iodophor solution to the still hot granular bentonite just after granulated or just after drying, it is made possible to eliminate the drying step. When the granular bentonite for supporting iodophor is wet, it may be dried by solar drying or hot-air drying.

According to another embodiment of the present invention, the toilet sand for pets is obtained by blending 100 parts by weight of the granular bentonite having a BET specific surface area of from 80 to 800 $m^2/g$ and a pore volume of from 0.2 to 2.3 ml/g with 0.1 to 10 parts by weight of a powder which comprises an inorganic porous powder having a BET specific surface area of from 80 to 800 $m^2/g$ and a pore volume of from 0.2 to 2.3 ml/g carrying the iodophor in an amount of from 0.05 to 20 parts by weight per 100 parts by weight of the above powder. In this case, there can be used any iodophor-carrying powder that was described in connection with the iodo-complex.

As the powder for carrying the iodophor, there can be used a fine crystalline synthetic zinc phyllosilicate having a large specific surface area, magnesium phyllosilicate, aluminum-containing zinc phyllosilicate, or aluminum-containing magnesium phyllosilicate to carry the iodophor, presenting great advantage as toilet sand for pets reinforcing both environmental sterilizing activity and deodoring activity. It is also allowable to blend the iodophor-carrying powder and the powder of the above-mentioned phyllosilicate.

The iodophor carrier and the granular bentonite can be mixed together by effecting the mixing to a small degree by using a mixer such as conical blender or drum blender.

When a silica gel is used as cat sand in the ammonia adsorption testing to examine deodoring, bacteria propagates on the silica gel and offensive odor is produced due to ammonia that is once adsorbed and due to ammonia emitted by bacteria. When pivodone is carried by silica gel, however, propagation of bacteria is suppressed, and production of offensive odor is prevented (see Example 11, Comparative Example 4).

[Thermoplastic polymer composition]

According to another embodiment of the present invention, there is provided a thermoplastic polymer composition obtained by blending a thermoplastic polymer or an elastomer with the above-mentioned iodo-complex; i.e., a thermoplastic polymer molded article having antibacterial activity is obtained.

As the thermoplastic polymer, there can be used a polyolefin such as random or block copolymer of $\alpha$-olefins like low-density polyethylene, high-density polyethylene, polypropylene, poly 1-butene, poly 4-methyl-1-pentene or ethylene, propylene, 1-butene or 4-methyl-1-pentene; an ethylene-vinyl compound copolymer such as ethylene-vinyl acetate copolymer, ethylene-vinyl alcohol copolymer or ethylene-vinyl chloride copolymer; a styrene resin such as polystyrene, acrylonitrile-styrene copolymer, ABS or $\alpha$-methylstyrene-styrene copolymer; a polyvinyl compound such as polyvinyl chloride, polyvinylidene chloride, vinyl chloride-vinylidene chloride copolymer, methyl polyacrylate or methyl polymethacrylate; a polyamide such as nylon 6, nylon 6-6, nylon 6-10, nylon 11 or nylon 12; a thermoplastic polyester such as polyethylene terephthalate or polybutylene terephthalate; a polycarbonate; a polyphenylene oxide, etc., or a mixture thereof. By using a biodegradable plastic, it is allowed to provide a resin molded article friendly to the environment, as a matter of course.

As the elastomer polymer, there can be used nitrile-butadiene rubber (NBR), styrene-butadiene rubber (SBR), chloroprene rubber (CR), polybutadiene (BR), polyisoprene (IIB), butyl rubber, natural rubber, ethylene-propylene rubber (EPR), ethylene-propylenediene rubber (EPDM), polyurethane, silicone rubber or acrylic rubber; or a thermoplastic elastomer such as styrene-butadiene-styrene block copolymer, styrene-isoprene-styrene block copolymer, hydrogenated styrene-butadiene-styrene block copolymer or hydrogenated styrene-isoprene-styrene block copolymer. Among them, hydrocarbon-type elastomer and, particularly, EPR and EPDM are particularly preferred.

It is desired that the iodo-complex is blended in an amount of from 0.1 to 30 parts by weight per 100 parts by weight of the thermoplastic resin or the elastomer. This makes it possible to obtain a molded polymer article having antibacterial activity. The thermoplastic resin may be directly kneaded with 0.01 to 20 parts by weight of povidone iodo accompanied, however, by poor dispersion. Besides, breeding takes place as the amount of addition increases. On the other hand, the iodo-complex of the present invention can be favorably blended in the polymer suppressing thermal degradation of both the iodo-complex and the polymer. The molded article of the polymer composition exhibits a variety of excellent properties.

The polymer can be blended with the iodo-complex by the so-called dry blending or the melt blending. Furthermore, the polymer may be blended with a master batch which contains the iodo-complex at a relatively high concentration.

It is desired that the kneading is effected at a relatively low temperature at which the polymer melts, i.e., at a temperature not higher than 200°C. To carry out the kneading at a relatively low temperature, it is desired to use a plasticizer; i.e., there can be used a plastisol such as the so-called vinyl chloride resin.

The polymer composition can be molded into films, sheets and pipes by the extrusion molding, into tanks and containers by the blow molding, into various housings and parts by the injection molding, and into sheets by the calender molding. The molded articles exhibit excellent antibacterial activity. It is, of course, allowable to effect the simultaneous extrusion or the simultaneous injection to obtain a molded polymer article of a laminated structure in which the surface layer only is blended with the iodo-complex.

The iodo-complex of the present invention may be blended in a widely known paint to use it as an antibacterial paint composition. Examples of the paint include, depending upon the kinds of resins, nitrocellulose paint, alkyd resin paint, aminoalkyd paint, vinyl resin paint, acrylic resin paint, epoxy resin paint, polyester resin paint, chlorinated rubber paint, as well as paints containing one or two or more of phenolic resin, modified phenolic resin, alkyd resin, vinyl resin, petroleum resin, epoxy resin, polyester resin, styrene resin, silicone resin, chlorinated compound resin, urethane resin, polyamide resin, polyimide resin and fluorine-containing resin.

Depending upon the use, furthermore, the paint may be a solvent-type paint, an aqueous paint, an ultraviolet-curing paint or a powder paint. According to the present invention, however, the solvent-type paint or the aqueous paint is particularly suited. The organic solvent for the solvent-type paint may be one or two or more of toluene, xylene, n-heptane, n-hexane, cyclohexane, acetone, methyl ethyl ketone, methylisobutyl ketone, cyclohexanone ethanol, propanol, butanol, diacetone alcohol tetrahydrofurane, dioxaneethyl cellosolve, butyl cellosolve ethyl acetate and dimethyl sulfoxide. As the aqueous paint, there can be used a self emulsifying paint or a surfactant emulsifying paint in addition to the aqueous solution-type paint. As the resin for the aqueous paint, there can be used an alkyd resin dissolved in an aqueous medium or is self-emulsified, a polyester resin, an acrylic resin or an epoxy resin in one kind or in a combination of two or more kinds. The resin concentration is usually from 10 to 70% by weight and, particularly, from 20 to 60% by weight.

Though it may vary depending upon the use, the iodo-complex of the present invention and, particularly, the spherical antibacterial agent is added in an amount of from 5 to 100 parts by weight per 100 parts by weight of the resin, and is used as an antibacterial paint.

[Antibacterial filter]

The above-mentioned iodo-complex can be used for the antibacterial filter such as the filter of an air cleaner.

For instance, MRSA dispersed in a hospital survives at least a week in a dry state, suspended in the air, diffuses, and is inhaled by many and unspecified persons who then may become carriers. In order to break the infection route in a hospital, the air in, for example, a concentrated treatment room is circulated through an air cleaner which is loaded with the antibacterial agent of the present invention to clean the contaminated air in the room. The antibacterial treatment is carried out by using, as a filter, the iodo-complex that is supported by a Raschig ring that was mentioned in the Section G) above.

Fig. 1 is a diagram for explaining an apparatus for testing the antibacterial activity of a filter for an air cleaner using the Raschig ring-type antibacterial agent, and wherein a container 3 is connected to the exhaust side of an air pump 1. The container 3 is filled with used animal-breeding chips 6, and has two pipes 2 connected thereto, one of the pipe being directly connected to a container containing a control culture medium 8. The other pipe is connected to a container containing a testing culture medium 9 through a container containing an antibacterial sample 7, i.e., the sample (GI-9). The containers containing the control culture medium 8 and the testing culture medium 9 are hermetically sealed with rubber plugs 4, and the exhaust tubes are fitted with cotton plugs 10 so that no bacteria will enter therein.

The air that has come into contact with the chips 6 also came in contact with the control culture medium 8, and where colonies of bacteria grew. On the other hand, even when the air that has come into contact with the Raschig ring of the present invention also came into contact with the testing culture medium, no colony of bacteria grew.

[Antibacterial sheet]

A sheet of at least one layer obtained by filling an air-permeable woven fabric or nonwoven fabric of inorganic fibers, animal and plant fibers or synthetic resin fibers with iodo-complex particles gradually releasing iodine of the present invention, can be used as an antibacterial sheet for such applications as filters or mats that will be described later. For instance, the iodo-complex-filled portion is sandwiched between the upper and lower fiber sheets to obtain an antibacterial sheet. It is also allowable to fill woven fabric, nonwoven fabric, paper and porous resin sheet with the iodo-complex to use them for such applications.

Fig. 2 is a perspective view for explaining the antibacterial sheet, and Fig. 3 is an A-A sectional view of the bacterial sheet. The antibacterial sheet consists of an upper sheet 12 having protruded and recessed portions, and a flat lower sheet 13. Iodo-complex particle-filling portions 14 are formed between the protruded portions 11 of the upper sheet 12 and the lower sheet 13. This embodiment uses a sample (GI-10).

The above-mentioned antibacterial sheet can be used for such applications as mat, carpet, sheet for beds in hospitals, floor material, wall material, etc., in addition to the filter.

Fig. 4 is a diagram illustrating how the antibacterial sheet is used, wherein an antibacterial sheet 17 is laid on a mattress 18 on a bed, an ordinary sheet 16 is stretched thereon and a patient 15 lies down thereon.

The antibacterial sheet of the present invention employs the iodo-complex that exhibits deodoring activity as described above. Therefore, deodoring activity is obtained while antibacterial activity is being imparted, and more desirable environment is created.

[Use]

As described above, the iodo-complex which gradually releases iodine of the present invention can be used for a variety of applications as described below, and as an antiseptic, anti-molding agent, deodoring agent as well as compositions thereof.

(1) Sterilizer for cattle sheds and machines for stock raising, e.g., for sterilizing floors, walls and ceilings of cowhouse, pigpen, henhouse, etc., for sterilizing milking machine, incubator, machines for diagnosing and reproducing cattle, and for sterilizing nipples after the milking.
(2) Sterilizer and antibacterial agent for machines for producing foods and for fermentation, e.g., for sterilizing facilities, machinery, warehouse of raw materials and products, refrigerators, containers and packaging materials, gloves and shoes of workers.
(3) Sterilizer for lumbers, e.g., for imported lumbers, lumbering, chips, plywoods, leathers, fibers and paints.
(4) Environmental sterilizer and antibacterial agent for sewage disposal facilities, excretions disposal facilities, conduit pipes and drain pipes, putrid swamps and ponds, etc.
(5) Sterilizer and antibacterial agent for public facilities, e.g., for sterilizing lavatories in a hospital, words and beds in a hospital, for sterilizing meal rooms in schools and kindergartens, lavatories and sandboxes, and for sterilizing meal centers, theaters, public lavatories, public bathhouses and vehicles.
(6) Sterilizer and antibacterial agent for plants, e.g., as an anti-molding agent and as an insecticidal agent for imported plants, tea leaves and crude drugs, and for sterilizing warehouses and refrigerators.
(7) Sterilizer and antibacterial agent for domestic use, e.g., as an antiseptic and anti-molding agent for bath rooms and bathtubs, for sterilizing washing machines, kitchen, sink, refrigerators, kitchen garbage, toilet stools, toilet bowls (for infants, aged people, patients), and for sterilizing pets and toilet sand for pets.

[EXAMPLES]

The invention will now be described in detail by way of Examples. The iodine compound which gradually releases iodine and the antibacterial agent used in the present invention were evaluated for their properties in a manner as described below.

(1) Apparent density.

Measured in compliance with JIS K-6220.6.8.

(2) Oil-absorbing amount.

Measured in compliance with JIS K-5101.19.

(3) pH.

Ten grams of the sample was added to 100 ml of the ion-exchanged water followed by stirring, which was then left to stand at 25°C for one hour, and the supernatant solution was measured for its pH value.

(4) Specific surface area, pore volume.

The specific surface area and the pore volume were measured in accordance with the BET method by using

Sorptomatic Series 1800 manufactured by Carlo Erba Co. By using a mercury intrusion-type porosimeter (Autopore 9220 manufactured by Micromeritics Co.), furthermore, pore sizes of from 18 to 43500 angstroms were measured to find the pore volume.

(5) Particle diameter.

The particle diameter was measured by using an aperture tube of 50 μm in compliance with the Coulter counter method (Model TA-II, manufactured by Coulter Electronics Co.).

(6) Particle diameter by SEM.

Representative particles were selected from a photograp obtained by using a scanning electron microscope (S-570 manufactured by Hitachi, Ltd.), long diameters and short diameters of particles were measured by using a scale, and were regarded to be primary particle diameters.

(7) Degree of sphericity.

Representative particles were selected from a photograph obtained by using the scanning electron microscope (S-570 manufactured by Hitachi, Ltd.), long diameters and short diameters of particles were measured using a scale, and the degree of sphericity was found in compliance with the following formula,

$$\text{Degree of sphericity} = \text{short diameter (DS)/long diameter (DL)}$$

(8) Rate of iodine elution.

A predetermined amount of iodo-complex carrying a predetermined amount of iodine ($I_2$) was taken into a column, the ion-exchanged water maintained at 20°C was permitted to flow at a rate of 5 ml/min., and a rate of iodine elution (meq/hr • 100 g) of the iodo-complex which gradually releases iodine of the present invention was calculated by regarding, as an end point, a moment at which the color of the povidone iodo extinguishes and no blue color due to the iodine-starch reaction is recognized in the effluent.

(9) Antibacterial testing.

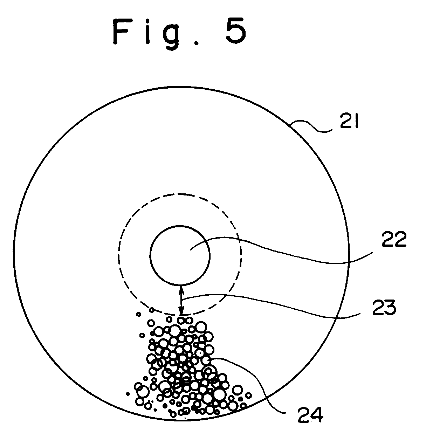

One milliliter of a solution obtained by suspending a strain to be tested in a physiological saline water at a concentration of about 1 x $10^6$ bacteria/ml was mixed into 2 ml of a culture medium which was maintained at 50°C and was comprised of 0.2% of peptone, 0.7% of table salt and 0.8% of agar. The mixture was then superposed on a flat agar culture medium having the same composition comprising 1.5% of agar. Then, the sample of the invention was placed on the superposed layer and was completely solidified. Then, the strain was cultured at 37°C for 24 hours, and the width of the propagation blocking band around the sample was measured (Fig. 5). Here, to the bacteria NIP4000 was further added glucose in an amount of 0.1% as a culture component.

(10) Amounts of effective iodine and iodide ions.

The sterilizing and antibacterial activities of the iodine compound are exhibited by iodine ($I_2$) itself. Therefore, the amount of ionic iodine and the amount of other effective iodine (iodine molecules) in the whole iodine in the sample must be evaluated by methods described below.

1. As for effective iodine, 2 g of the sample powder was suspended in 30 ml of water, and the amount % of effective iodine (Ia) was determined by the iodine-starch reaction titration using a 0.005N solution of sodium thiosulfate.
2. As for the iodide ions, 2 g of the sample powder was suspended in 100 ml of water, a solution of sodium hydrogensulfite was added thereto until the color of iodine disappeared, 10 ml of a 0.1N silver nitrate solution and 4 ml of nitric acid were added thereto, and the whole amount % of iodine (I) was found by the titration with a 0.05N ammonium thiocyanate solution (indicator: ammonium ferric sulfate) using an excess amount of silver nitrate. A difference (I) - (Ia) represents the amount (%) of iodide ions.

(Example 1)

Described below are a variety of iodo-complexes gradually releasing iodine according to the present invention.

10 Parts by weight of a powder of povidone iodo (manufactured by Nippo Kagaku Co.) was added as an iodine compound (B) to 100 parts by weight of each of the powdery carriers (A), i.e., amorphous Mizukasorb (produced by Mizusawa Kagaku Co., having a specific surface area of 400 $m^2/g$, a porous volume of 1.5 ml, a pH of 5) which is a porous and amorphous silica, Silton A (produced by Mizusawa Kagaku Co., having a specific surface area of 250 $m^2/g$, a pore volume of 0.3 ml/g, a pH of 5.3), acidic clay Silton LP of 2-octahedral smectite (produced by Mizusawa Kagaku Co., having a specific surface area of 90 $m^2/g$, a pore volume of 0.2 ml/g, a pH of 5), and Mizukalife of magnesium phyllosilicate of 3-octahedral smectite (produced by Mizusawa Kagaku Co., having a specific surface area of 650 $m^2/g$, a pore volume of 0.8 ml/g, a pH of 8.5). The mixtures were wetted with water, kneaded together, and were granulated into cylinders (pellets having a diameter of 1.5 mm) by the extrusion granulation method, dried under a condition of 60 to 120°C to obtain iodo-complexes which gradually release iodine of the present invention (PI-1, PI-2, PI-3, PI-4).

To PI-1 and PI-3 were further added, as organic surface treating agents, a silicone oil and PEG-800 placed in the market in amounts of 20 and 10 parts by weight per 100 parts by weight of the total of (A) and (B) at the time of kneading, to obtain samples PI-1S and PI-3S.

Similarly, 100 parts by weight of each of the carriers (A), i.e., Mizupearl in the form of definite spherical particles (produced by Mizuwasa Kagaku Co., having an average particle diameter of 3 μm, a specific surface area of 500 $m^2/g$, a pore volume of 1.0 ml/g, a pH of 7), MS silica (produced by Mizuwasa Kagaku Co., having an average particle diameter of 30 μm, a specific surface area of 350 $m^2/g$, a pore volume of 1.5 ml/g, a pH of 7.5), fine spherical silica (produced by Mizusawa Kagaku Co., having an average particle diameter of 3 mm, a specific surface area of 750 $m^2/g$, a pore volume of 1.1 ml/g, a pH of 5), and fine spherical silica (produced by Mizusawa Kagaku Co., having an average particle diameter of 1 mm, a specific surface area of 300 $m^2/g$, a pore volume of 1.5 ml/g) were impregnated with an aqueous solution containing 10% of the pividone iodine (produced by Nippo Kagaku Co.) that corresponds to 10 parts by weight of povidone iodine solid component as the iodine compound (B), followed by the treatment in the same manner as described above to obtain spherical iodo-complexes that gradually release iodine of the present invention (samples Nos. SI-5S (silicon oil), SI-6S (PEG-800), SI-7S (silicon oil), SI-8S (PEG-800)). After impregnated with povidone iodine, the samples were rolled and treated on their surfaces with the silicone oil and PEG-800, followed by drying at 60 to 90°C.

By using part of the spherical iodo-complex SI-6S which gradually releases iodine and using a powder molding machine (Correct D555-C15R) which is a compression (tabletting) molding machine manufactured by Kikusui Mfg. Co., there was prepared a molded iodo-complex gradually releasing iodine of the type of Raschig ring having an outer diameter of 5 mm, an inner diameter of 2 mm and a height of 6 mm (sample No. GI-9).

(Example 2)

In order to evaluate the ability for gradually releasing iodine of the iodo-complexes of the present invention, agar was added in an amount of 1.5% and soluble starch was added in an amount of 0.1% to water and were completely dissolved therein. The solution was then introduced into a laboratory dish to a thickness of about 3 to 4 mm, and was left to cool and solidify. On the agar were then placed PI-1, PI-2, PI-3, PI-3S, SI-5S, SI-6S, SI-8S obtained in Example 1 and a powder of povidone iodine for the purpose of comparison. Blue color due to the iodine-starch reaction lasted for several days around the samples of the present invention. However, blue color around the powder of the povidone iodine disappeared the next day. It was therefore learned that the iodo-complexes of the present invention gradually released iodine in a sustained manner.

(Example 3)

Iodo-complexes gradually releasing iodine of samples Nos. PI-1, PI-2, PI-3, PI-4, PI-1S, PI-3S, SI-5S, SI-6S, SI-7S, SI-8S and GI-9 obtained in Example 1 were measured for their amounts (%) of effective iodine and amounts (%) of iodide ions, and were tested for their antibacterial activities against Staphylococcus aureus NIP 2019 (MRSA), Pseudomonas aeruginosa IFM 3011 and Candida albicans NIP 4000, and against IFM 2058, IFM 3039, IFM 3029, IFM 3041 and IFM3048. The results of antibacterial testing were as shown in Tables 1 and 2. Here, MRSA and IFM 3011 are bacteria immune to antibiotics and are arousing a problem of hospital infection, and NIP 4000 is a pathogenic yeast causing opportunistic infection.

It will be observed that the bacteria are propagating forming colonies around the blocking bands on the agar culture medium. IFM 2058, IFM 3039, IFM 3029 and IFM 3041 are food poisoning bacilli and IFM 3048 is a dysentery bacillus.

Table 1

| Sample No. | Amount of effective iodine (%) | Species/Width of blocking band (mm) | | |
|---|---|---|---|---|
| | | MRSA | Pseudomonas aeruginosa | Candida albicans |
| PI-1 | 0.68 | 10 | 1 | 3 |
| PI-2 | 0.72 | 10 | 1 | 2 |
| PI-3 | 0.74 | 10 | 2 | 4 |
| PI-1S | 0.58 | 8 | 2 | 4 |
| PI-3S | 0.62 | 12 | 2 | 4 |
| PI-5S | 0.51 | 9 | 1 | 2 |
| PI-6S | 0.82 | 12 | 2 | 3 |
| PI-7S | 0.65 | 10 | 2 | 3 |
| PI-8S | 0.64 | 11 | 1 | 2 |
| GI-9 | 0.79 | 11 | 2 | 2 |

Table 2

| Sample No. | Amount of effective iodine (%) | Species/Width of blocking band (mm) | | | | |
|---|---|---|---|---|---|---|
| | | IFM 2058 | IFM 3039 | IFM 3029 | IFM 3041 | IFM 3048 |
| (food poisoning bacilli ) | (dysentery bacillus) | | | | | |
| PI-1 | 0.68 | 9 | 4 | 4 | 3 | 2 |
| PI-2 | 0.72 | 10 | 3 | 4 | 4 | 4 |
| PI-3 | 0.74 | 11 | 4 | 3 | 3 | 4 |
| PI-1S | 0.58 | 8 | 2 | 4 | 2 | 2 |
| PI-3S | 0.62 | 9 | 3 | 3 | 4 | 2 |
| PI-5S | 0.51 | 9 | 2 | 3 | 4 | 3 |
| PI-6S | 0.82 | 11 | 4 | 4 | 4 | 4 |
| PI-7S | 0.65 | 10 | 3 | 3 | 2 | 2 |
| PI-8S | 0.64 | 8 | 3 | 3 | 3 | 2 |
| GI-9 | 0.79 | 10 | 4 | 4 | 4 | 4 |

The rates of iodine elution ISV of the samples are PI-1 (0.37), PI-2 (0.40), PI-3 (2.3), PI-4 (0.36), PI-1S (0.18), PI-3S (0.20), PI-5S (0.08), PI-6S (0.15), PI-7S (0.02), and PI-8S. (0.17).

(Example 4)

By using an air pump (blow rate of 1.5 l/min.), the air was blown into a container packed with muck of animal breeding, and the air from the container was passed through a column (carrier length of 43 cm, inner diameter of 25 mm) filled with the sample GI-9. The air from the column was passed through a container containing on the bottom thereof the Brain Heart Infusion agar culture medium for two days. As a comparison, the air from the container packed

with the same muck was directly passed in parallel with the container containing on the bottom thereof the Brain Heart Infusion agar culture medium. After the air was passed, the containers containing the culture media were left to stand under aerobic conditions at 37°C for two days. Not less than 500 colonies of bacteria grew on the comparative culture medium, whereas only ten colonies grew on the tested culture medium. Fig. 1 shows a testing apparatus.

(Example 5)

The ethylene-vinyl acetate copolymer resin (EVA) was blended with the sample SI-5S in amounts as described below, and was stirred and kneaded (Henschel's mixer). The obtained kneaded products were supplied to a biaxial extruder and were pelletized at a temperature of 150°C, and were then subjected to the inflation molding to obtain films having a width of 250 mm and a thickness of 100 μm.

EVA 100 parts : SI-5S 10 parts
(film sample 5-1)
EVA 100 parts : SI-5S 5 parts
(film sample 5-2)
EVA 100 parts only
(film sample 5-3)

Table 3 shows the films and the results of the antibacterial testing.

(Example 6)

A sheet obtained by three-dimensionally working nonwoven fabric was filled with a material (GI-10) comprising 100 parts of sepiolite carrying 10 parts of pivodone iodine to obtain a sheet (filling amount, 800 $g/m^2$, produced by Nichiei Kogyo Co.: Korukemi sheet) (see Figs. 2 and 3).

The flat surface of the sheet was faced upwards, and 2 ml of a sample of bacillus pyocyaneus (Pseudomonas aeruginosa) or MRSA was put thereon and was permitted to be absorbed by the mat for one hour. Then, a filter paper was spread under the sheet and was pushed from the upper side using a spatula, so that the sample absorbed by the sheet was caused to ooze out onto the filter paper. The filter paper was stuck to a standard agar culture medium for detecting bacteria and was left overnight. The filter paper was peeled, and the agar culture medium was cultured at 37°C for 24 hours to detect bacteria. As results, the sample solution of bacillus pyocyaneus or MRSA adsorbed by the mat containing polyvinyl pyrrolidone had been sterilized. Even when the solution was forcibly oozed out, no bacteria was detected. In the comparative sheet (using sepiolite alone), on the other hand, the solution had not been sterilized despite it was absorbed by the sheet. When the sample solution that had been absorbed was forcibly oozed out, the bacteria leaked and could be detected on the agar culture medium.

(Example 7)

The antibacterial sheet used in Example 6 was spread on the mats or the beds used by aged persons of from 71 to 80 years old (3 men and 2 women) who were suffering from chronic diseases and were treated in the homes, and sheets were spread thereon (see Fig. 4). The antibacterial sheets were replaced by new ones once a month. The sheets were used for three months to clinically evaluate whether bedsore could be prevented.

As for the bedsore, 2 persons were already developing bedsore to a slight degree and one person was developing bedsore to an intermediate degree among 5 persons. Table 4 shows the results of observation of bedsore of five persons after every month, and Table 5 shows the results of deodoring test.

The degrees of bedsore were as follows:

intermediate degree: ++
slight degree: +
no bedsore: -

The references of evaluating the deodoring test was as follows:

++: strong odor
+: weak odor
-: no odor

Moreover, the following testing was conducted in order to make sure whether the mat was soaked with MRSA. The sheet that was used and the surface of the mat were cut into suitable sizes and were rinsed with a suitable amount of physiological saline water. An agar culture medium of yolk-added mannitol table salt to which has been added 4 μg/ml of oxacillin (MPIPC) and an agar culture medium of yolk-added mannitol table salt were smeared with 0.1 ml of the solutions diluted with physiological saline solution into 10 times of steps, and were aerobically cultured at 37°C for 40 hours. Among the colonies, those described below were regarded to be MRSA, and were qualitatively cultured. The results were as shown in Table 6.

Confirmation of MRSA: gram positive coccus based on gram dyeing, coagulase positive, mannitol decomposition, yolk reaction positive, resistant to 7.5% table salt.
resistant to 4 μg/ml of oxacillin in addition to the above-mentioned properties

Table 3

| Species (cm) | | MRSA blocking band width | Pseudomonas artuginosa blocking band width |
|---|---|---|---|
| Film sample | 5-1 | 4.5 | 3 |
| | 5-2 | 3.0 | 2 |
| | 5-3 | 0 | 0 |

Table 4

| Clinical test of bedsore. | | | | |
|---|---|---|---|---|
| Patient | Before use | 1 Month after | 2 Months after | 3 Months after |
| A | + | + | + | - |
| B | + | + | + | - |
| C | ++ | ++ | ++ | + |
| D | - | - | - | - |
| E | - | - | - | - |
| Medium degree: ++<br>Slight degree: +<br>None: - | | | | |

Table 5

| Deodoring test | | | |
|---|---|---|---|
| | | At the start of testing | 2 Weeks after |
| Patient | A | ++ | + |
| | B | ++ | + |
| | C | + | - |
| | D | - | - |
| | E | - | - |

++: Strong odor
+: Weak odor
-: No odor

Table 6

| Detection of MRSA | | | |
|---|---|---|---|
| | | Number of bacteria | |
| | | MRSA detected in the sheet | MRSA detected on the surface of mat |
| patient | A | $1 \times 10^4/100\ cm^2$ | not detected |
| | B | $8 \times 10^4/100\ cm^2$ | not detected |
| | C | $3 \times 10^5/100\ cm^2$ | not detected |
| | D | not detected | not detected |
| | E | not detected | not detected |

In the following examples, measurements were taken as described below.

(1) Testing the antibacterial activity.

Eleven bacterial strains were tested including gram negative bacilli such as Escherichia coli F1 (colon bacillus), Pseudomonas aeruginosa IFM3011 (bacillus pyocyaneus), Shigella flexneri IFM3046 (dysentery bacillus), Salmonella enteritidis IFM3029 (salmonella), Klebsiella oxytocia IFM3046 (klebsiella), Vibrio parahaemolyticus IFM3014 (enteritis vibrio), Campylobacter sp. No. 2 (campylobacter); gram positive coccuci such as Staphylococcus aureus MFI2014 (xanthostaphylococcus), Methicillin-resistant Staphylococcus aureus NIP2019 (MRSA, methicillin-resistant xanthostaphylococcus), Enterococcus faecalis IFM2001 (enterococcus); and yeast such as Candida albicans IFM40083 (candida).

The bacterial solution was prepared by culturing bacteria overnight in the Brain Heart Infusion culture medium (produced by Difco Co., hereinafter abbreviated as BHI, 3% NaCl was added to enteritis vibrio), the bacteria were washed one time (using a centrifuge revolving at 3000 rpm for 15 minutes) and were suspended again in a PBS (to one liter of purified water were added 3.26 g of $KH_2PO_4$, 0.4 g of $Na_2HPO_4$, and 5.1 g of NaCl, pH being adjusted to be 6.0). Camphylobacter was aerobically cultured for two days using a BHI agar culture medium in accordance with a gas-pack method (manufactured by BBL Co.), and candida was aerobically cultured for two days in a potato dextrose agar culture medium (PDA). The colonies (bacteria) that grew were washed one time with PBS, and were suspended again in the PBS. These bacterial solutions were suspended in the PBS so that the number of live bacteria was finally $10^6$ to $10^7$/ml.

One gram of toilet sand for pets was added to 10 ml of each of the bacterial solutions prepared as described above and was left to stand at room temperature (about 25°C) for 30 minutes to measure the number of live bacteria. As a comparative example, the treatment was carried out in the same manner in the PBS without adding no toilet sand for pets. The number of live bacteria was measured as described below. That is, to the solution to be tested was added an equal amount of an aqueous solution of 1% of sodium thiosulfate to inactivate iodine that is present. Then, by using the BHI agar culture medium (3% of NaCl was added to enteritis vibrio) or PDA for candida in a customary manner, the number of live bacteria was calculated from the number of colonies that grew after culturing.

(2) Testing the deodoring effect.

100 Grams of the sample was introduced into a one-litter sealed glass container with a silicone rubber plug to collect gases, and to which was dropwisely added 20 ml of urea collected from a cat of three years old. After left to stand at room temperature for 1, 7, 15 and 30 days, the concentration of ammonia gas was measured using a gas detector tube.

(3) Testing the solidification.

The sample was introduced into a tall 300-ml beaker to a depth of 6 cm. 7 Milliliters of a 1% saline solution which is a substitute of pet urea was dropwisely added thereto from the upper side over a period of 10 seconds to solidify the sample and to evaluate the solidified state.

Described below are references for evaluation:

◎: Does not easily collapse even when a pressure is given thereto by fingers.
○: Collapses when a pressure is given thereto by fingers.
Δ: Remains solid but collapses when picked up by hand.
X: Not solidified.

(Example 8)

First step.

Sodium carbonate in an amount of 3.0% by weight reckoned as an anhydride thereof was added to 5 kg of water-containing smectite clay. The mixture was mixed together using a mixer and was granulated into grains of a diameter of 3 mm and a length of about 7 mm using a biaxial extrusion kneader to obtain smectite (activated bentonite) molded articles.

Second step.

A solution obtained by dissolving 1 g of a polyvinyl pyrrolidone-iodine complex (produced by Nippo Kagaku Co.) in 20 g of a water-alcohol mixture solution (15 g of ion-exchanged water and 5 g of ethanol), was uniformly sprayed onto 1000 g of the molded products obtained in the first step using a paint spray gun (Winder 71 manufactured by Iwata Kogyo Co.) to obtain toilet sand for pets.
The toilet sand for pets obtained through the second step was subjected to the antibacterial testing, deodoring testing and solidification testing. The results were as shown in Tables 7, 8 and 9.

(Example 9)

First step.

The polyvinyl pyrrolidone-iodine complex (produced by Nippo Kagaku Co.) was added in an amount of 10% by weight to 1 kg of water-containing smectite clay. The mixture was mixed together using a mixer, and was further kneaded and granulated using a biaxial extrusion kneader and a die of a diameter of 3 mm. The thus molded articles were then dried at 80°C using a dryer and were granulated into particles of a diameter of 3 mm and a length of about 7 mm.

Second step.

100 Grams of the molded articles obtained through the first step and 900 g of the smectite molded articles obtained through the first step of Example 1 were homogeneously mixed together to obtain toilet sand for pets.
The toilet sand for pets obtained through the second step was subjected to the antibacterial testing, deodoring testing and solidification testing. The results were as shown in Tables 7, 8 and 9.

(Example 10)

First step.

One kilogram of a fraiponite-silica complex (Mizukanite, produced by Mizusawa Kagaku Kogyo Co.) and 50 g of the polyvinyl pyrrolidone-iodine complex were mixed together followed by the addition of 800 g of ion-exchanged water. The mixture was mixed together using a mixer. The mixture was then kneaded and granulated using a biaxial extrusion kneader and a dies of a diameter of 3 mm. The molded articles were then dried using a dryer and were granulated into particles of a diameter of 3 mm and a length of about 7 mm.

Second step.

100 Grams of the molded articles obtained through the first step and 900 g of the smectite molded articles obtained through the first step of Example 1 were homogeneously mixed together to obtain toilet sand for pets.
The toilet sand for pets obtained through the second step was subjected to the antibacterial testing, deodoring testing and solidification testing. The results were as shown in Tables 7, 8 and 9.

(Example 11)

First step.

500 Grams of silica gel placed in the market (Silbead, produced by Mizusawa Kagaku Kogyo Co., having a BET specific area of 450 $m^2/g$ and an $N_2$-adsorbing pore volume of 1.5 ml/g) and 2 g of the polyvinyl pyrrolidone-iodine complex (passing 100 mesh) were introduced into a 2-liter pot mill, and were mixed together being revolved at 60 rpm for 10 minutes to obtain toilet sand for pets.

The thus obtained toilet sand for pets was subjected to the antibacterial testing, deodoring testing and solidification testing. The results were as shown in Tables 7, 8 and 9.

(Example 12)

First step.

Sodium carbonate was added in an amount of 150 g reckoned as an anhydride to 5 kg of water-containing smectite clay (water-containing acidic clay). The mixture was mixed together using a mixer, and to which was further added 500 g of aluminum sulfate (20% reckoned as $Al_2O_3$). The mixture was then molded into particles having a diameter of 3 mm and a length of 7 mm using a biaxial extrusion kneader.

Second step.

A solution obtained by dissolving 1 g of the polyvinyl pyrrolidone-iodine complex (povidone iodo) in 20 g of a water-alcohol mixture solution (15 g of ion-exchanged water and 5 g of ethanol) was uniformly sprayed onto 1000 g of the molded articles obtained through the first step using a paint spray gun (Wonder 71 manufactured by Iwata Kogyo Co.) to obtain toilet sand for pets.

The toilet pets obtained through the second step was subjected to the antibacterial testing, deodoring testing and solidification testing. The results were as shown in Tables 7, 8 and 9.

(Example 13)

To 100 parts by weight of the toilet sand for pets obtained in Example 8 was mixed 20 parts by weight of the molded products of activated bentonite and aluminum sulfate obtained in the first step of Example 12 to prepare toilet sand for pets.

The toilet sand for pets was subjected to the antibacterial testing, deodoring testing and solidification testing. The results were as shown in Tables 7, 8 and 9.

(Example 14)

First step.

8 Kilograms of acidic clay (about 6 kg in dry form) and 30 liters of water were wet-pulverized in a ball mill for 2 hours to obtain a wet-pulverized slurry. To 30 kg of the slurry (about 4.5 kg in dry form) obtained by further adding water to the above slurry was added 2.16 kg of an aluminum sulfate solution ($Al_2O_3$ content of 7.58% by weight), which was then stirred and reacted for one hour and was then dry-pulverized at 110°C for 15 hours. The thus acid-treated smectite was molded into particles of a diameter of 3 mm and a length of 7 mm using a biaxial extrusion mixer.

Second step.

A solution obtained by dissolving 1 g of the polyvinyl pyrrolidone-iodine complex (povidone iodo) in 20 g of a water-alcohol mixture solution (15 g of ion-exchanged water and 5 g of ethanol) was uniformly sprayed onto 1000 g of the molded articles obtained through the first step using a paint spray gun (Wonder 71 manufactured by Iwata Kogyo Co.) to obtain toilet sand for pets.

The toilet pets obtained through the second step was subjected to the antibacterial testing, deodoring testing and solidification testing. The results were as shown in Tables 7, 8 and 9.

(Example 15)

To 100 parts by weight of the toilet sand for pets obtained in Example 8 were added 10 parts by weight of the aluminum-sulfate-containing acid-treated smectite obtained through the first step of Example 14 and 10 parts by weight of the acid-treated montmorillonite to obtain toilet sand for pets.
The toilet sand for pets was subjected to the antibacterial testing, deodoring testing and solidification testing. The results were as shown in Tables 7, 8 and 9.

(Comparative Example 1)

Natural bentonite-type cat sand placed in the market was subjected to the antibacterial testing, deodoring testing and solidification testing. The results were as shown in Tables 7, 8 and 9.

(Comparative Example 2)

Natural zeolite-type cat sand placed in the market was subjected to the antibacterial testing, deodoring testing and solidification testing. The results were as shown in Tables 7, 8 and 9.

(Comparative Example 3)

Natural pulp-type cat sand placed in the market was subjected to the antibacterial testing, deodoring testing and solidification testing. The results were as shown in Tables 7, 8 and 9.

(Comparative Example 4)

Natural silica gel-type cat sand placed in the market was subjected to the antibacterial testing, deodoring testing and solidification testing. The results were as shown in Tables 7, 8 and 9.

Table 7 (Results of antibacterial testing)

| Bacteria to be detected | Number of live bacteria (/ml) after 10 min. | | | | | | | | | | | | | |
|---|---|---|---|---|---|---|---|---|---|---|---|---|---|---|
| | Initially | PBS only | Ex. 8 | Ex. 9 | Ex. 10 | Ex. 11 | Ex. 12 | Ex. 13 | Ex. 14 | Ex. 15 | Comp. Ex. 1 | Comp. Ex. 2 | Comp. Ex. 3 | Comp. Ex. 4 |
| Escherichia coli F1 | $3.6 \times 10^6$ | $3.0 \times 10^6$ | ND* | ND | ND | ND | ND | ND | ND | ND | $2.9 \times 10^6$ | $2.8 \times 10^6$ | $3.2 \times 10^6$ | $2.5 \times 10^6$ |
| Pseudomonas aeruginosa IFM3011 | $2.7 \times 10^7$ | $3.0 \times 10^7$ | ND | ND | ND | ND | ND | ND | ND | ND | $2.3 \times 10^7$ | $2.8 \times 10^7$ | $3.0 \times 10^7$ | $3.1 \times 10^7$ |
| Shigella flexneri IFM3046 | $5.0 \times 10^7$ | $4.6 \times 10^7$ | ND | ND | ND | ND | ND | ND | ND | ND | $4.7 \times 10^7$ | $4.9 \times 10^7$ | $5.0 \times 10^7$ | $4.3 \times 10^7$ |
| Salmonella enteritidis IFM3029 | $2.5 \times 10^7$ | $2.9 \times 10^7$ | ND | ND | ND | ND | ND | ND | ND | ND | $2.1 \times 10^7$ | $2.3 \times 10^7$ | $2.7 \times 10^7$ | $2.3 \times 10^7$ |
| Klebsiella oxytoca IFM3046 | $2.3 \times 10^7$ | $2.8 \times 10^7$ | ND | ND | ND | ND | ND | ND | ND | ND | $2.5 \times 10^7$ | $2.8 \times 10^7$ | $2.1 \times 10^7$ | $2.0 \times 10^7$ |
| Vibrio parahaemolyticus IFM3014 | $1.1 \times 10^7$ | $1.0 \times 10^7$ | ND | ND | ND | ND | ND | ND | ND | ND | $1.0 \times 10^7$ | $9.1 \times 10^6$ | $9.5 \times 10^6$ | $9.9 \times 10^6$ |
| Campylobacter sp. Kitazato labo.No.2 | $1.6 \times 10^8$ | $1.1 \times 10^8$ | ND | ND | ND | ND | ND | ND | ND | ND | $1.3 \times 10^8$ | $9.8 \times 10^7$ | $9.3 \times 10^7$ | $1.0 \times 10^8$ |
| Staphylococcus aureus IFM2014 | $7.4 \times 10^6$ | $5.2 \times 10^6$ | ND | ND | ND | ND | ND | ND | ND | ND | $6.1 \times 10^6$ | $7.0 \times 10^6$ | $5.1 \times 10^6$ | $4.3 \times 10^6$ |
| MRSA NIP2019 | $1.1 \times 10^7$ | $1.3 \times 10^7$ | ND | ND | ND | ND | ND | ND | ND | ND | $1.0 \times 10^7$ | $9.2 \times 10^6$ | $9.0 \times 10^6$ | $1.1 \times 10^7$ |
| Enterococcus faecalis IFM2001 | $2.2 \times 10^7$ | $2.1 \times 10^7$ | ND | ND | ND | ND | ND | ND | ND | ND | $1.9 \times 10^7$ | $1.1 \times 10^7$ | $1.5 \times 10^7$ | $2.0 \times 10^7$ |
| Candida albicans IFM40083 | $4.5 \times 10^6$ | $4.1 \times 10^6$ | ND | ND | ND | ND | ND | ND | ND | ND | $4.1 \times 10^6$ | $3.5 \times 10^6$ | $3.9 \times 10^6$ | $4.3 \times 10^6$ |

ND: Not detected (detectable limit is 20 bacteria/ml)

Table 8

| Concentration of ammonia gas (ppm) | | | | |
|---|---|---|---|---|
| | After 1 day | After 7 days | After 15 days | After 30 days |
| Example 8 | 3 | 3 | 5 | 5 |
| Example 9 | 2 | 2 | 3 | 3 |
| Example 10 | 0 | 0 | 0 | 0 |
| Example 11 | 0 | 0 | 0 | 0 |
| Example 12 | 0 | 0 | 0 | 0 |
| Example 13 | 0 | 0 | 0 | 0 |
| Example 14 | 0 | 0 | 0 | 0 |
| Example 15 | 0 | 0 | 0 | 0 |
| Comp. Ex. 1 | 3 | 12 | 19 | 20 |
| Comp. Ex. 2 | 3 | 8 | 15 | 18 |
| Comp. Ex. 3 | 10 | 16 | 19 | 22 |
| Comp. Ex. 4 | 0 | 5 | 9 | 13 |

Table 9

| Results of solidification testing | |
|---|---|
| Example 8 | ◎ |
| Example 9 | ◎ |
| Example 10 | ◎ |
| Example 11 | X |
| Example 12 | △ |
| Example 13 | ◎ |
| Comp. Ex. 1 | ○ |
| Comp. Ex. 2 | X |
| Comp. Ex. 3 | △ |
| Comp. Ex. 4 | X |

## Claims

1. An iodo-complex having a sustained release ability for iodine, comprising (A) an inorganic carrier having a BET specific surface area of from 80 to 800 $m^2$/g and a pore volume of from 0.2 to 2.3 ml/g as measured by the nitrogen adsorption method, and (B) an iodophor carried by said inorganic carrier.

2. An iodo-complex according to claim 1, wherein said inorganic carrier (A) is a porous and amorphous silica powder.

3. An iodo-complex according to claim 2, wherein said inorganic carrier (A) has a pore volume with a pore radius of from 7.5 to 150 angstroms of from 20 to 90% per the whole pore volume.

4. An iodo-complex according to claim 1, wherein said inorganic carrier (A) is a powder of porous and activated alumina or activated silica-alumina.

5. An iodo-complex according to claim 1, wherein said inorganic carrier (A) is a phyllosilicate or a phylloaluminosilicate.

6. An iodo-complex according to claim 1, wherein said inorganic carrier (A) is a powder of clay minerals of the smectite group or acid-treated clay minerals of the smectite group.

7. An iodo-complex according to claim 1, wherein said inorganic carrier (A) is a powder of bentonite or a mixture of bentonite and aluminum sulfate.

8. An iodo-complex according to claim 1, wherein said inorganic carrier (A) is a powder of fraiponite or a porous and amorphous silica carrying fraiponite on the surfaces thereof.

9. An iodo-complex according to claim 1, wherein said inorganic carrier (A) is a powder of clay minerals of the hormite type.

10. An iodo-complex according to any one of claims 1 to 9, wherein said inorganic carrier (A) has an average particle diameter of from 0.0001 to 4 mm.

11. An iodo-complex according to claim 1, wherein said inorganic carrier (A) is a porous molded article of at least one kind selected from the group consisting of amorphous silica, activated alumina, activated silica-alumina, clay minerals of the smectite group, fraiponite and clay minerals of the hormite type.

12. An iodo-complex according to claim 1, wherein said inorganic carrier (A) comprises amorphous and porous spherical silica particles having a volume-based median diameter of from 10 to 100 $\mu$m as measured by using the Coulter counter, an apparent density (in compliance with JIS K-6220) of from 0.2 to 0.5 ml/g, and a pore volume with a pore radius of from 18 to 43500 angstroms of from 0.5 to 3.5 ml/g as measured by the mercury intrusion porosity method.

13. An iodo-complex according to claim 12, wherein said spherical and amorphous silica particles have meso pores of a pore radius of from 75 to 1000 angstroms as measured by the mercury intrusion porosity method and a pore volume of not smaller than 0.2 ml/g.

14. An iodo-complex according to claim 1, wherein said inorganic carrier (A) is an amorphous silica in the form of spherical particles having a primary particle diameter of from 0.2 to 20 $\mu$m as observed using a scanning electron microscope, the individual particles being obviously spherical particles having a degree of sphericity of not smaller than 0.9, and further having an apparent density (in compliance with JIS K-6220) of from 0.1 to 0.5 ml/g, a pore volume of from 0.5 to 2 ml/g as measured by the nitrogen adsorption method, and an oil-absorbing amount of from 100 to 400 ml/100 g.

15. An iodo-complex according to claim 1, wherein said iodophor (B) contains effective iodine in an amount of from 5 to 20% by weight.

16. An iodo-complex according to claim 1, wherein said iodophor (B) is povidone iodo.

17. An iodo-complex according to claim 1, wherein the iodophor (B) is contained in an amount of from 0.05 to 30 parts by weight per 100 parts by weight of said inorganic carrier (A).

18. An iodo-complex having a sustained release ability for iodine, comprising (A) an inorganic carrier having a BET specific surface area of from 80 to 800 $m^2$/g and a pore volume of from 0.2 to 2.3 ml/g as measured by the nitrogen adsorption method, (B) an iodophor carried by said inorganic carrier, and (C) a covering layer for covering them and comprising at least one of oil, wax or resin.

19. An iodo-complex according to claim 18, wherein the iodine compound (B) is contained in an amount of from 0.05 to 30 parts by weight per 100 parts by weight of said inorganic carrier (A), and the covering layer is contained in an amount of from 1 to 30 parts by weight per 100 parts by weight of the total of (A) and (B).

20. An antibacterial agent comprising the iodo-complex having a sustained release ability for iodine of any one of claims 1 to 19.

21. Toilet sand for pets containing the iodo-complex having a sustained release ability for iodine of any one of claims 1 to 19.

22. A filter for an air cleaner containing the iodo-complex having a sustained release ability iodine of any one of claims 1 to 19.

23. A thermoplastic polymer composition obtained by blending a thermoplastic resin or an elastomer with the iodo-complex having a sustained release ability for iodine of any one of claims 1 to 19.

24. A thermoplastic polymer composition according to claim 23, wherein the iodo-complex is blended in an amount of from 0.1 to 30 parts by weight per 100 parts by weight of the thermoplastic resin or the elastomer.

25. A carpet comprising a thermoplastic polymer composition of claim 23 or 24.

26. An antibacterial filter comprising at least one layer of sheet obtained by filling an air-permeable woven fabric or nonwoven fabric of inorganic fibers, animal fibers, plant fibers, or synthetic resin fibers with the iodo-complex of any one of claims 1 to 19.

27. An antibacterial mat comprising at least one layer of sheet obtained by filling a woven fabric or nonwoven fabric of inorganic fibers, animal fibers, plant fibers, or synthetic resin fibers with the iodo-complex of any one of claims 1 to 19.

## Patentansprüche

1. Jodkomplex mit Depotwirkung für Jod umfassend (A) einen anorganischen Träger mit einer spezifischen Oberfläche nach BET von 80 bis 800 $m^2/g$ und einem Porenvolumen von 0,2 bis 2,3 ml/g, gemessen nach der Stickstoffadsorptionsmethode, und (B) einen vom anorganischen Träger getragenen Jodophor.

2. Jodkomplex nach Anspruch 1, dadurch gekennzeichnet, daß der anorganische Träger (A) ein poröses und amorphes Siliciumdioxid-Pulver ist.

3. Jodkomplex nach Anspruch 2, dadurch gekennzeichnet, daß der anorganische Träger (A) ein Porenvolumen mit einem Porenradius von 7,5 bis 150 Ångström in einer Menge von 20 bis 90 % des gesamten Porenvolumens aufweist.

4. Jodkomplex nach Anspruch 1 dadurch gekennzeichnet, daß der anorganische Träger (A) ein Pulver aus porösem und aktiviertem Aluminiumoxid oder aktiviertem Siliciumdioxid-Aluminiumoxid ist.

5. Jodkomplex nach Anspruch 1, dadurch gekennzeichnet, daß der anorganische Träger (A) ein Phyllosilicat oder ein Phylloalumosilicat ist.

6. Jodkomplex nach Anspruch 1, dadurch gekennzeichnet, daß der anorganische Träger (A) ein Pulver aus Tonmineralien der Smektit-Gruppe oder von säurebehandelten Tonmineralien der Smektit-Gruppe ist.

7. Jodkomplex nach Anspruch 1, dadurch gekennzeichnet, daß der anorganische Träger (A) ein Pulver aus Bentonit oder einer Mischung aus Bentonit und Aluminiumsulfat ist.

8. Jodkomplex nach Anspruch 1, dadurch gekennzeichnet, daß der anorganische Träger (A) ein Pulver aus Fraiponit oder einem porösen und amorphem Siliciumdioxid, das an seinen Oberflächen Fraiponit aufweist, ist.

9. Jodkomplex nach Anspruch 1, dadurch gekennzeichnet, daß der anorganische Träger (A) ein Pulver aus Tonmineralien des Hormit-Typs ist.

10. Jodkomplex nach einem der Ansprüche 1 bis 9, dadurch gekennzeichnet, daß der anorganische Träger (A) einen mittleren Teilchendurchmesser von 0,0001 bis 4 mm aufweist.

**11.** Jodkomplex nach Anspruch 1, dadurch gekennzeichnet, daß der anorganische Träger (A) ein poröser Formkörper mindestens einer Art ist, die ausgewählt ist aus der Gruppe bestehend aus amorphen Siliciumdioxid, aktiviertem Aluminiumoxid, aktiviertem Siliciumdioxid-Aluminiumoxid, Tonmineralien der Smektit-Gruppe, Fraiponit und Tonmineralien des Hormit-Typs.

**12.** Jodkomplex nach Anspruch 1, dadurch gekennzeichnet, daß der anorganische Träger (A) amorphe und poröse kugelförmige Siliciumdioxidteilchen mit einem mittleren Durchmesser (Volumenbasis) von 10 bis 100 µm, gemessen unter Verwendung eines Coulter-Zählers, eine scheinbare Dichte (gemäß JIS K-6220) von 0,2 bis 0,5 ml/g, ein Porenvolumen mit einem Porenradius von 18 bis 43500 Ångström von 0,5 bis 3,5 ml/g, gemessen nach der Quecksilberintrusions-Porösitätsbestimmungsmethode, aufweist.

**13.** Jodkomplex nach Anspruch 12, dadurch gekennzeichnet, daß die kugelförmigen und amorphen Siliciumdioxidteilchen Mesoporen mit einem Porenradius von 75 bis 1000 Ångström, gemessen nach der Quecksilberintrusions-Porösitätsbestimmungsmethode, und einem Porenvolumen von nicht kleiner als 0,2 ml/g aufweisen.

**14.** Jodkomplex nach Anspruch 1, dadurch gekennzeichnet, daß der anorganische Träger (A) ein amorphes Siliciumdioxid in Form von kugelförmigen Teilchen mit einem primären Teilchendurchmesser von 0,2 bis 20 µm, bestimmt unter Verwendung eines Rasterelektronenmikroskops, ist, die individuellen Teilchen kugelförmige Teilchen mit einem Sphärizitätsgrad von nicht kleiner als 0,9 sind, und außerdem eine scheinbare Dichte (gemäß JIS K-6220) von 0,1 bis 0,5 ml/g, ein Porenvolumen von 0,5 bis 2 ml/g, gemessen nach der Stickstoffadsorptionsmethode, und ein Ölabsorptionsvermögen von 100 bis 400 ml/100g besitzen.

**15.** Jodkomplex nach Anspruch 1, dadurch gekennzeichnet, daß der Jodophor (B) wirksames Jod in einer Menge von 5 bis 20 Gew.-% enthält.

**16.** Jodkomplex nach Anspruch 1, dadurch gekennzeichnet, daß der Jodophor (B) Povidon-Jod ist.

**17.** Jodkomplex nach Anspruch 1, dadurch gekennzeichnet, daß der Jodophor (B) in einer Menge von 0,05 bis 30 Gewichtsteile pro 100 Gewichtsteile des anorganischen Trägers (A) enthalten ist.

**18.** Jodkomplex mit Depotwirkung für Jod umfassend (A) einen anorganischen Träger mit einer spezifischen Oberfläche nach BET von 80 bis 800 $m^2/g$ und einem Porenvolumen von 0,2 bis 2,3 ml/g, gemessen nach der Stickstoffadsorptionsmethode, (B) einen vom anorganischen Träger getragenen Jodophor, und (C) eine Deckschicht, um diese abzudecken, die mindestens ein Öl, Wachs oder Harz umfaßt.

**19.** Jodkomplex nach Anspruch 18, dadurch gekennzeichnet, daß die Jodverbindung (B) in einer Menge von 0,05 bis 30 Gewichtsteile pro 100 Gewichtsteile des anorganischen Trägers (A) enthalten ist, und die Deckschicht in einer Menge von 1 bis 30 Gewichtsteilen pro 100 Gewichtsteile von (A) und (B) zusammen enthalten ist.

**20.** Antibakterielles Mittel umfassend den Jodkomplex mit Depotwirkung für Jod nach einem der Ansprüche 1 bis 19.

**21.** Toilettensand für Haustiere enthaltend den Jodkomplex mit Depotwirkung für Jod nach einem der Ansprüche 1 bis 19.

**22.** Filter für einen Luftreiniger enthaltend den Jodkomplex mit Depotwirkung für Jod nach einem der Ansprüche 1 bis 19.

**23.** Thermoplastische Polymerzusammensetzung, erhalten durch Mischen eines thermoplastischen Harzes oder eines Elastomers mit dem Jodkomplex mit Depotwirkung für Jod nach einem der Ansprüche 1 bis 19.

**24.** Thermoplastische Polymerzusammensetzung nach Anspruch 23, dadurch gekennzeichnet, daß der Jodkomplex in einer Menge von 0,1 bis 30 Gewichtsteilen pro 100 Gewichtsteile des thermoplastischen Harzes oder des Elastomers zugemischt ist.

**25.** Teppich umfassend eine thermoplastische Polymerzusammensetzung nach Anspruch 23 oder 24.

**26.** Antibakterielles Filter umfassend mindestens eine Schicht einer Bahn, die erhalten wurde durch Füllen eines luftdurchlässigen Textilgewebes oder Faservlieses aus anorganischen Fasern, tierischen Fasern, Pflanzenfasern oder

synthetischen Harzfasern mit dem Jodkomplex nach einem der Ansprüche 1 bis 19.

27. Antibakterielle Matte umfassend mindestens eine Schicht einer Bahn, die erhalten wurde durch Füllen eines Textilgewebes oder Faservlieses aus anorganischen Fasern, tierischen Fasern, Pflanzenfasern oder synthetischen Harzfasern mit dem Jodkomplex nach einem der Ansprüche 1 bis 19.

**Revendications**

1. Complexe d'iode présentant une aptitude à la libération prolongée d'iode, comprenant (A) un support minéral ayant une surface spécifique BET de 80 à 800 $m^2/g$ et un volume de pores de 0,2 à 2,3 ml/g, tel que mesuré par le procédé d'adsorption d'azote, et (B) un iodophore porté par ledit support minéral.

2. Complexe d'iode selon la revendication 1, dans lequel ledit support minéral (A) est une poudre de silice poreuse et amorphe.

3. Complexe d'iode selon la revendication 2, dans lequel ledit support minéral (A) a un volume de pores avec un rayon de pores de 7,5 à 150 angströms de 20 à 90% du volume de pores total.

4. Complexe d'iode selon la revendication 1, dans lequel ledit support minéral (A) est une poudre d'alumine activée et poreuse ou de silice-alumine activée.

5. Complexe d'iode selon la revendication 1, dans lequel ledit support minéral (A) est un phyllosilicate ou un phylloaluminosilicate.

6. Complexe d'iode selon la revendication 1, dans lequel ledit support minéral (A) est une poudre de minéraux argileux du groupe smectite ou de minéraux argileux traités par un acide du groupe smectite.

7. Complexe d'iode selon la revendication 1, dans lequel ledit support minéral (A) est une poudre de bentonite ou un mélange de bentonite et de sulfate d'aluminium.

8. Complexe d'iode selon la revendication 1, dans lequel ledit support minéral (A) est une poudre de fraiponite ou une silice poreuse et amorphe portant de la fraiponite sur ses surfaces.

9. Complexe d'iode selon la revendication 1, dans lequel ledit support minéral (A) est une poudre de minéraux argileux du groupe hormite.

10. Complexe d'iode selon l'une quelconque des revendications 1 à 9, dans lequel ledit support minéral (A) présente un diamètre particulaire moyen de 0,0001 à 4 mm.

11. Complexe d'iode selon la revendication 1, dans lequel ledit support minéral (A) est un article moulé poreux d'au moins un type choisi dans le groupe constitué par la silice amorphe, l'alumine activée, la silice-alumine activée, des minéraux argileux du groupe smectite, la fraiponite et des minéraux argileux du groupe hormite.

12. Complexe d'iode selon la revendication 1, dans lequel ledit support minéral (A) comprend des particules sphériques de silice amorphe et poreuse ayant un diamètre moyen en volume de 10 à 100 $\mu m$, tel que mesuré en utilisant le compteur Coulter, une masse volumique apparente (conformément à la norme JIS K-6220) de 0,2 à 0,5 ml/g, et un volume de pores avec un rayon de pores de 18 à 43500 angströms de 0,5 à 3,5 ml/g, tel que mesuré par le procédé de porosité par introduction de mercure.

13. Complexe d'iode selon la revendication 12, dans lequel lesdites particules sphériques de silice amorphe et poreuse ont des mésopores présentant un rayon de pores de 75 à 1000 angströms, tel que mesuré par le procédé de porosité par introduction de mercure et un volume de pores qui n'est pas inférieur à 0,2 ml/g.

14. Complexe d'iode selon la revendication 1, dans lequel ledit support minéral (A) est une silice amorphe sous forme de particules sphériques ayant un diamètre de particule primaire de 0,2 à 20 $\mu m$ tel qu'observé en utilisant un microscope électronique à balayage, les particules individuelles, étant évidemment sphériques, ayant un degré de sphéricité qui n'est pas inférieur à 0,9, et ayant en outre une masse volumique apparente (conformément à la norme JIS K-6220) de 0,1 à 0,5 ml/g, un volume de pores de 0,5 à 2 ml/g, tel que mesuré par le procédé d'adsorption d'azote, et une capacité d'absorption d'huile de 100 à 400 ml/100 g.

**15.** Complexe d'iode selon la revendication 1, dans lequel ledit iodophore (B) contient de l'iode actif en une quantité de 5 à 20% en poids.

**16.** Complexe d'iode selon la revendication 1, dans lequel ledit iodophore (B) est un complexe povidoneiode.

**17.** Complexe d'iode selon la revendication 1, dans lequel l'iodophore (B) est présent en une quantité de 0,05 à 30 parties en poids pour 100 parties en poids dudit support minéral (A).

**18.** Complexe d'iode présentant une aptitude à la libération prolongée d'iode, comprenant (A) un support minéral ayant une surface spécifique BET de 80 à 800 $m^2/g$ et un volume de pores de 0,2 à 2,3 ml/g, tel que mesuré par le procédé d'adsorption d'azote, (B) un iodophore porté par ledit support minéral, et (C) une couche de revêtement pour les recouvrir et comprenant au moins une huile, une cire ou une résine.

**19.** Complexe d'iode selon la revendication 18, dans lequel ledit composé d'iode (B) est présent en une quantité de 0,05 à 30 parties en poids pour 100 parties en poids dudit support minéral (A), et la couche de revêtement est présente en une quantité de 1 à 30 parties en poids pour 100 parties en poids du total de (A) et de (B).

**20.** Agent antibactérien comprenant le complexe d'iode présentant une aptitude à la libération prolongée d'iode selon l'une quelconque des revendications 1 à 19.

**21.** Litière pour animaux domestiques contenant le complexe d'iode présentant une aptitude à la libération prolongée d'iode selon l'une quelconque des revendications 1 à 19.

**22.** Filtre pour épurateur d'air contenant le complexe d'iode présentant une aptitude à la libération prolongée d'iode selon l'une quelconque des revendications 1 à 19.

**23.** Composition de polymère thermoplastique obtenue en mélangeant une résine thermoplastique ou un élastomère avec le complexe d'iode présentant une aptitude à la libération prolongée d'iode selon l'une quelconque des revendications 1 à 19.

**24.** Composition de polymère thermoplastique selon la revendication 23, dans laquelle le complexe d'iode est mélangé en une quantité de 0,1 à 30 parties en poids pour 100 parties en poids de la résine thermoplastique ou de l'élastomère.

**25.** Tapis comprenant une composition de polymère thermoplastique selon la revendication 23 ou 24.

**26.** Filtre antibactérien comprenant au moins une couche d'une feuille obtenue en chargeant un textile tissé ou un textile non tissé, perméable à l'air, en fibres minérales, en fibres animales, en fibres végétales, ou en fibres de résine synthétique avec le complexe d'iode selon l'une quelconque des revendications 1 à 19.

**27.** Natte antibactérienne comprenant au moins une couche d'une feuille obtenue en chargeant un textile tissé ou un textile non tissé en fibres minérales, en fibres animales, en fibres végétales, ou en fibres de résine synthétique avec le complexe d'iode selon l'une quelconque des revendications 1 à 19.

Fig. 1

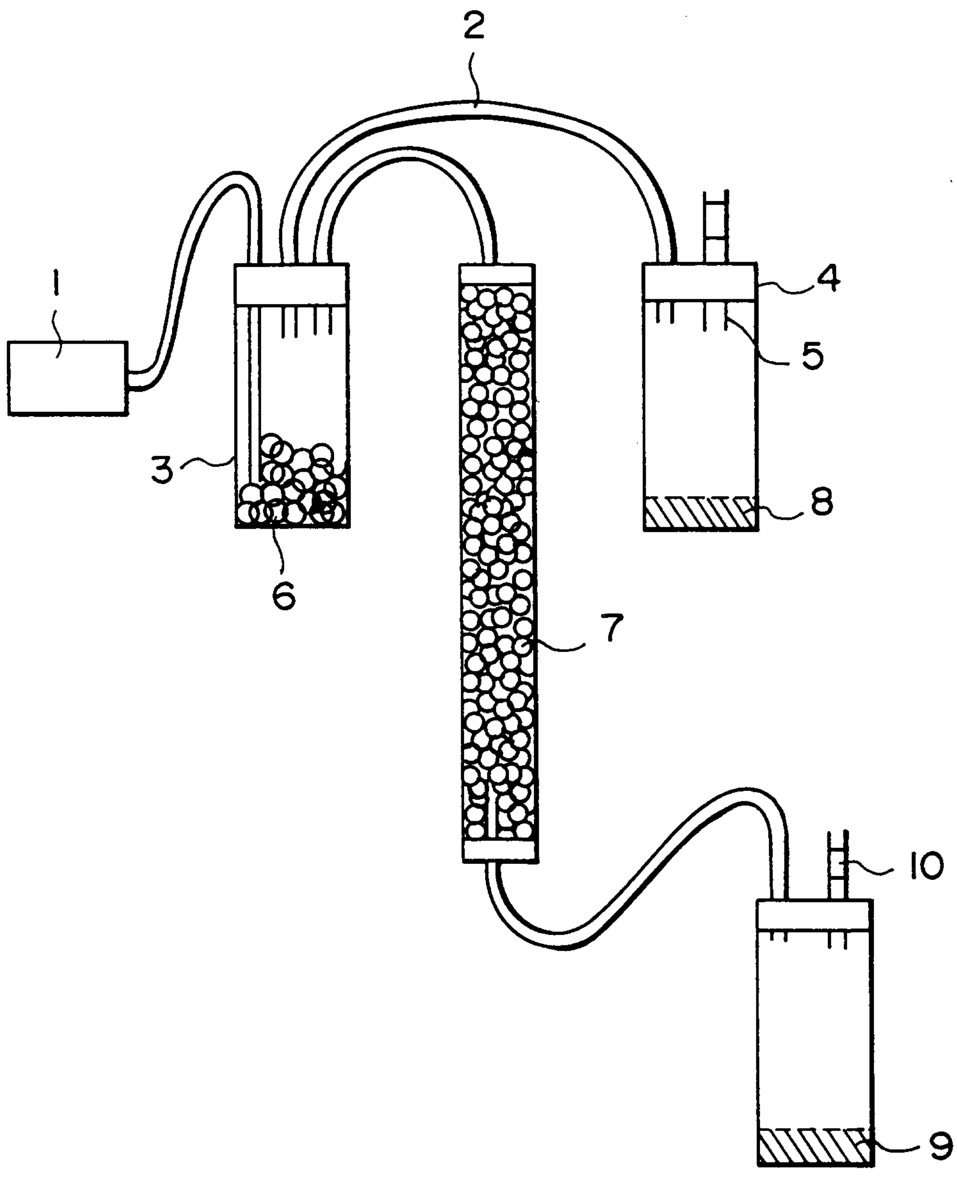

Fig. 2

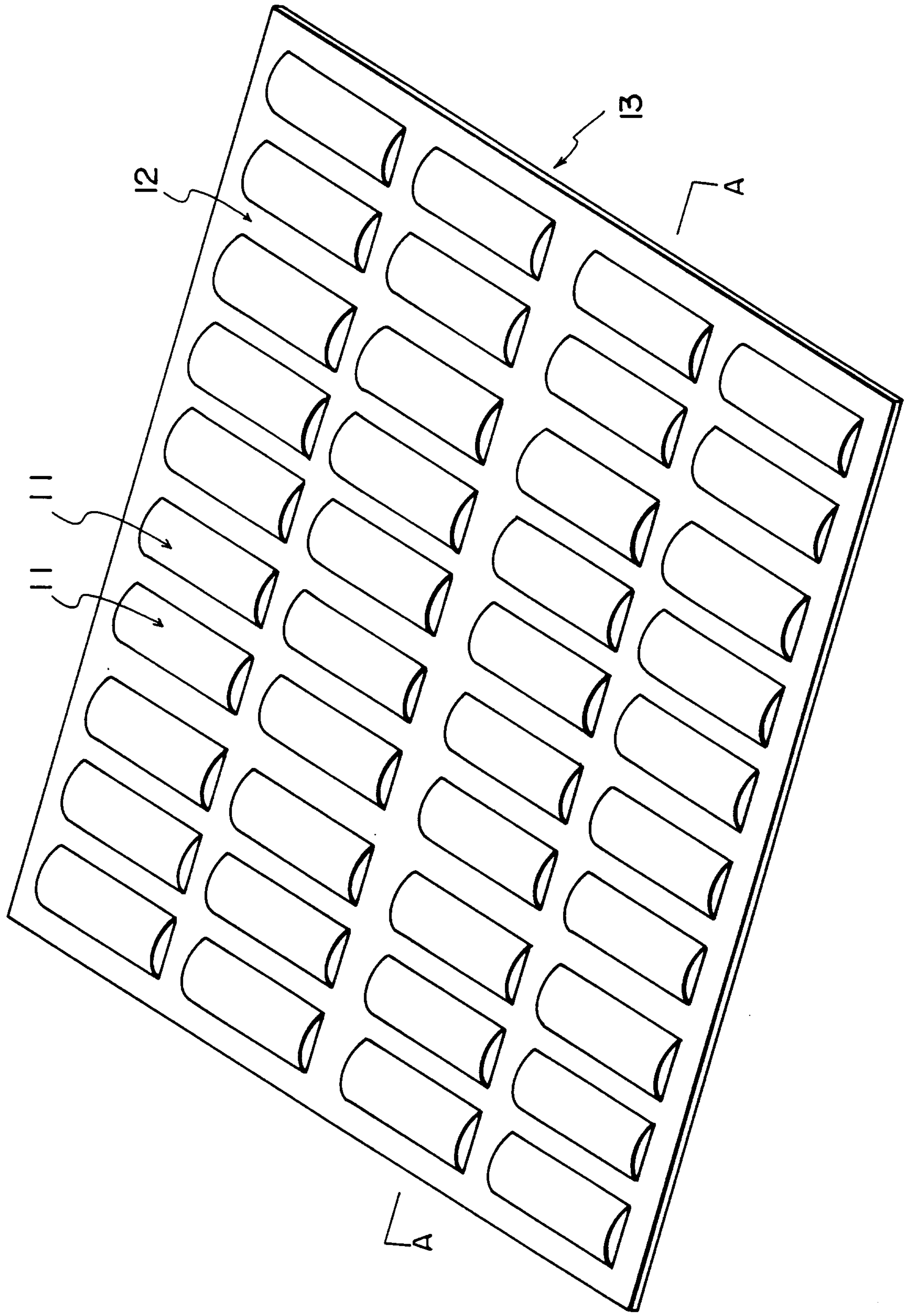

Fig. 3

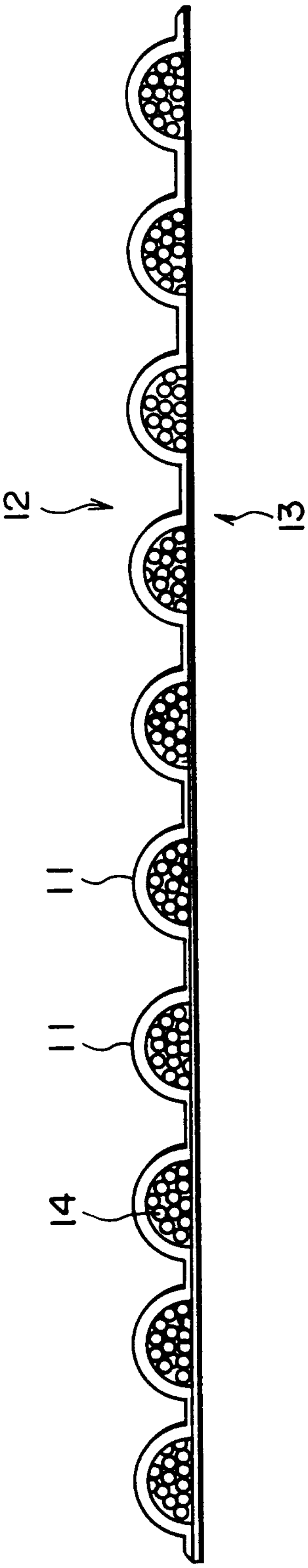

Fig. 4

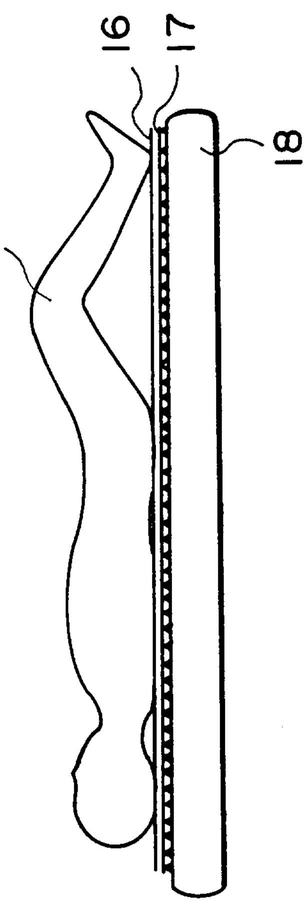

Fig. 5